# EUROPEAN PATENT APPLICATION

(11) **EP 4 693 306 A1**
(43) Date of publication of application: **11.02.2026**
(21) Application number: 24779268.2
(22) Date of filing: 07.03.2024
(51) Int. Cl.: G16C 20/30

(54) **TOXICITY EVALUATION DEVICE, METHOD FOR OPERATING TOXICITY EVALUATION DEVICE, AND PROGRAM FOR OPERATING TOXICITY DEVICE**

(30) Priority: 31.03.2023 JP 2023059324
(71) Applicant: FUJIFILM Corporation, Tokyo 106-8620 (JP)
(72) Inventor: HARA IMAMURA, Mika, Ashigarakami-gun, Kanagawa 258-8577 (JP); TAKAKU, Koji, Ashigarakami-gun, Kanagawa 258-8577 (JP); FUJITA, Masaharu, Ashigarakami-gun, Kanagawa 258-8577 (JP); TATESHITA, Masakazu, Ashigarakami-gun, Kanagawa 258-8577 (JP); HIKIDA, Yasushi, Ashigarakami-gun, Kanagawa 258-8577 (JP)
(74) Representative: HGF
(86) International application number: PCT/JP2024/008879
(87) International publication number: WO 2024/203134

(57) **Abstract**

A toxicity evaluation device including a processor, in which the processor is configured to perform data acquisition processing of acquiring test substance data specifying a test substance, prediction processing of predicting a metabolic reaction process of the test substance and a metabolite generated in the metabolic reaction process based on the test substance data, evaluation processing of acquiring an evaluation result by evaluating toxicity of at least one compound selected from the group consisting of the test substance and the metabolite, and output processing of outputting a processing result including the evaluation result, in the prediction processing, the processor is configured to predict the metabolic reaction process based on the test substance data and rule data specifying a rule in which a metabolic reaction proceeds, and the rule data includes a compatibility rule suitable for an in vitro toxicity evaluation test; an operation method of a toxicity evaluation device; and an operation program of a toxicity evaluation device.

## Description

### BACKGROUND OF THE INVENTION

### 1. Field of the Invention

The present disclosure relates to a toxicity evaluation device, an operation method of a toxicity evaluation device, and an operation program of a toxicity device.

### 2. Description of the Related Art

In recent years, in a field of handling chemical substances, toxicity evaluation using an in silico method has been performed. In the toxicity evaluation of a chemical substance, it is required to evaluate not only the toxicity of the chemical substance to be tested but also the toxicity of metabolites, to determine the toxicity to a living body.

Various in silico methods for predicting the metabolism or toxicity of a chemical substance have been developed so far. For example, J. Chem. Inf. Comput. Sci. 2003, 43, 1371 describes a model that predicts a metabolite of a compound without causing an excessive metabolic reaction. Chem. Res. Toxicol. 2004, 17, 753 describes a toxicity prediction model in a TA100 strain used in an Ames test, which is a type of toxicity test, taking into account liver metabolism. SAR and QSAR in Environmental Research, Vol. 22, Nos. 7-8, October-December 2011, 699-718 describes a model that mathematically associates the metabolic prediction with the toxicity result. Computational Toxicology 22 (2022) 100218 describes a model that optimizes the likelihood of metabolic transformation based on metabolic reaction data and toxicity data.

### SUMMARY OF THE INVENTION

However, many of the conventional models for predicting metabolism or toxicity are not suitable for predicting the in vitro toxicity evaluation test result or have insufficient prediction accuracy. For example, the model described in J. Chem. Inf. Comput. Sci. 2003, 43, 1371 is a model in which an excessive metabolic reaction does not occur, but since the relevance to the in vitro toxicity evaluation test is not taken into consideration, the prediction accuracy of the in vitro toxicity evaluation test is low. In the models described in Chem. Res. Toxicol. 2004, 17, 753, SAR and QSAR in Environmental Research, Vol. 22, Nos. 7-8, October-December 2011, 699-718, and Computational Toxicology 22 (2022) 100218, although the metabolic reaction rate is set based on the toxicity test results for the literature data on in vivo and in vitro metabolite analysis, the prediction accuracy of the in vitro toxicity evaluation test is not sufficient because the relevance to the compound that can be determined as toxic in the in vitro toxicity evaluation test is not sufficiently considered.

In view of the above situation, the present disclosure relates to a toxicity evaluation device, an operation method of a toxicity evaluation device, and an operation program of a toxicity device, which exhibit high prediction accuracy even in toxicity evaluation by an in silico method.

A toxicity evaluation device according to the technology of the present disclosure is a toxicity evaluation device including a processor, in which the processor is configured to perform data acquisition processing of acquiring test substance data specifying a test substance, prediction processing of predicting a metabolic reaction process of the test substance and a metabolite generated in the metabolic reaction process based on the test substance data, evaluation processing of acquiring an evaluation result by evaluating toxicity of at least one compound selected from the group consisting of the test substance and the metabolite, and output processing of outputting a processing result including the evaluation result, in the prediction processing, the processor is configured to predict the metabolic reaction process based on the test substance data and rule data specifying a rule in which a metabolic reaction proceeds, and the rule data includes a compatibility rule suitable for an in vitro toxicity evaluation test.

It is preferable that the test substance data is selected from the group consisting of a structural formula of the test substance, a compositional formula of the test substance, and an amino acid sequence of the test substance.

It is preferable that the rule data includes at least one condition selected from the group consisting of a reaction precondition that defines a premise under which a metabolic reaction occurs in the in vitro toxicity evaluation test, a reaction stop condition that defines a condition for stopping the metabolic reaction in the in vitro toxicity evaluation test, and a priority for preferentially handling a metabolic reaction that is likely to occur in the in vitro toxicity evaluation test, and the at least one condition selected from the group consisting of the reaction precondition, the reaction stop condition, and the priority includes the compatibility rule.

It is preferable that the priority is a priority given for each type of the metabolic reaction, and the priority includes a priority determined based on at least one reaction rate selected from the group consisting of a reaction rate by a metabolic enzyme used in the in vitro toxicity evaluation test and a reaction rate by a non-enzymatic reaction.

It is preferable that the compatibility rule includes a rule obtained from a relationship between a structure of a compound that has been subjected to toxicity evaluation in a past in vitro toxicity evaluation test and a metabolic mode estimated from a toxicity determination result.

It is preferable that the prediction processing includes a reaction prediction processing of predicting a metabolic reaction that is repeatedly performed stepwise starting from the test substance and a metabolite generated by the metabolic reaction at each stage.

It is preferable that the rule data is defined for each type of the metabolic reaction, and the processor is configured to execute, based on the rule data, the reaction prediction processing for each of the stages and each of the metabolic reactions.

It is preferable that in the reaction prediction processing, a maximum value of the number of stages of a metabolic reaction that is repeatedly performed stepwise starting from the test substance or the metabolite is limited based on a structure of the test substance or the metabolite.

It is preferable that in the reaction prediction processing, in a case where a plurality of predictions of the metabolic reaction occurring in one of the stages starting from the test substance or the metabolite are possible, limitation on the number of predictions of the metabolic reaction in the stage is possible.

It is preferable that the compatibility rule includes a priority for preferentially handling a metabolic reaction that is likely to occur in the in vitro toxicity evaluation test, and the limitation on the number of predictions is performed based on the priority.

It is preferable that in a case where a stage of a metabolic reaction occurring in the test substance is defined as a first stage and a stage of a metabolic reaction occurring in the metabolite generated by the metabolic reaction in the first stage is defined as a second stage, the limitation on the number of predictions based on the priority is performed for the metabolic reactions in the second stage and subsequent stages.

It is preferable that the compatibility rule includes a reaction precondition that defines a premise under which a metabolic reaction occurs in the in vitro toxicity evaluation test, a reaction stop condition that defines a condition for stopping the metabolic reaction in the in vitro toxicity evaluation test, and a priority for preferentially handling a metabolic reaction that is likely to occur in the in vitro toxicity evaluation test, and the limitation on the number of predictions is performed by limiting a prediction of the metabolic reaction occurring in one of the stages to a metabolic reaction in which a difference in priority from a metabolic reaction having the highest priority among a plurality of metabolic reactions not satisfying the reaction stop condition and satisfying the reaction precondition is within a preset range.

It is preferable that the preset range is less than 5.

It is preferable that in the output processing, the processor is configured to further output metabolic process information specifying the metabolic reaction process.

It is preferable that the in vitro toxicity evaluation test is an evaluation test for mutagenicity, sensitization, or irritation.

An operation method of a toxicity evaluation device according to the technology of the present disclosure is an operation method of a toxicity evaluation device including a processor, in which the processor is configured to perform: data acquisition processing of acquiring test substance data specifying a test substance to be evaluated; prediction processing of predicting a metabolic reaction process of the test substance and a metabolite generated in the metabolic reaction process based on the test substance data; evaluation processing of acquiring an evaluation result by evaluating toxicity of at least one compound selected from the group consisting of the test substance and the metabolite; and output processing of outputting a processing result including the evaluation result, in the prediction processing, the processor is configured to predict the metabolic reaction process based on the test substance data and rule data specifying a rule in which a metabolic reaction proceeds, and the rule data includes a compatibility rule suitable for an in vitro toxicity evaluation test result.

An operation program of a toxicity evaluation device according to the technology of the present disclosure performs: data acquisition processing of acquiring test substance data specifying a test substance to be evaluated; prediction processing of predicting a metabolic reaction process of the test substance and a metabolite generated in the metabolic reaction process based on the test substance data; evaluation processing of acquiring an evaluation result by evaluating toxicity of at least one compound selected from the group consisting of the test substance and the metabolite; and output processing of outputting a processing result including the evaluation result, in the prediction processing, the processor is configured to predict the metabolic reaction process based on the test substance data and rule data specifying a rule in which a metabolic reaction proceeds, and the rule data includes a compatibility rule suitable for an in vitro toxicity evaluation test result.

According to the present disclosure, there is provided a toxicity evaluation device, an operation method of a toxicity evaluation device, and an operation program of a toxicity device, which exhibit high prediction accuracy even in toxicity evaluation by an in silico method.

### BRIEF DESCRIPTION OF THE DRAWINGS

FIG. 1 is a diagram showing an outline of a toxicity evaluation device.
FIG. 2 is a block diagram showing an example of a hardware configuration of a toxicity evaluation device.
FIG. 3 is a diagram showing an example of a flow of data acquisition processing, prediction processing, and evaluation processing.
FIG. 4 is a diagram showing an outline of rule data.
FIG. 5 is a diagram conceptually showing an example of a metabolic pathway predicted in prediction processing.
FIG. 6 is a diagram conceptually showing an example of a metabolic pathway predicted in prediction processing.
FIG. 7 is a diagram conceptually showing an example of a metabolic pathway predicted in prediction processing.
FIG. 8 is a diagram conceptually showing an example of a metabolic pathway predicted in prediction processing.
FIG. 9 is a flowchart showing an example of a processing procedure of the toxicity evaluation device.
FIG. 10 is a flowchart showing an example of a processing procedure of prediction processing of the toxicity evaluation device.
FIG. 11 is a flowchart showing an example of a reaction prediction processing procedure of the toxicity evaluation device.
FIG. 12 is a flowchart showing an example of display of a processing result.

### DESCRIPTION OF THE PREFERRED EMBODIMENTS

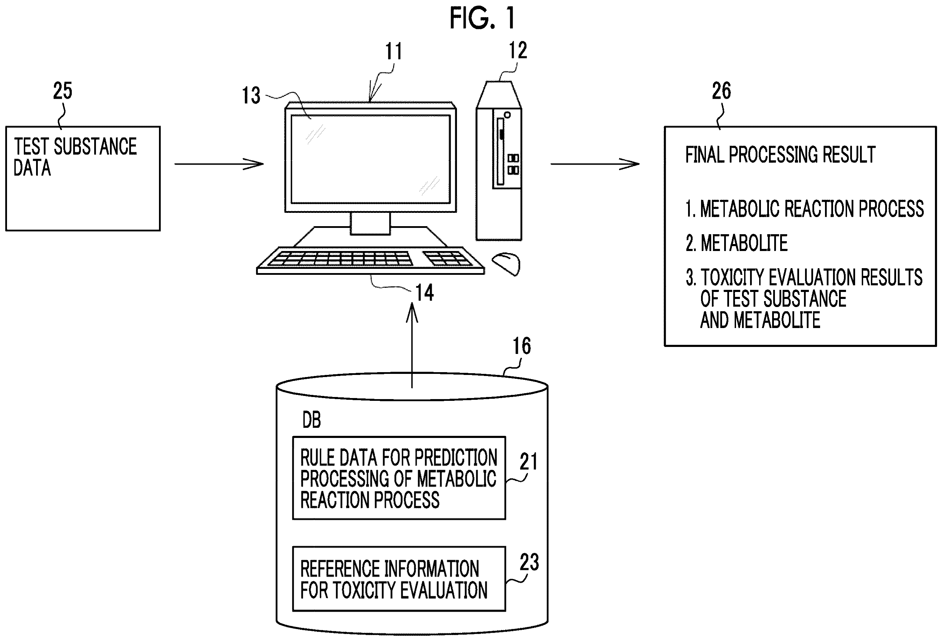

In FIG. 1, the toxicity evaluation device 11 is a device that evaluates the toxicity of a compound and outputs a processing result including an evaluation result. The toxicity evaluation device 11 is, for example, a desktop personal computer, and is operated by a user such as a researcher who performs the toxicity evaluation of the compound.

Test substance data 25 specifying the test substance is input to the toxicity evaluation device 11. The toxicity evaluation device 11 predicts a metabolic reaction process of a test substance and a metabolite generated in the metabolic reaction process. Then, the toxicity of at least one compound selected from the group consisting of the test substance and the metabolite is evaluated. The toxicity evaluation device 11 outputs a final processing result 26 including a metabolic reaction process, a metabolite, and a toxicity evaluation result of the test substance. The final processing result 26 is displayed on the display 13.

A body part 12, a display 13, an input device 14, and a database 16 (hereinafter, referred to as a DB) are connected to the toxicity evaluation device 11. The input device 14 is an operation unit such as a keyboard, a touch panel, and a mouse. DB 16 is, for example, constituted by a server computer or the like that delivers data in response to a request from the toxicity evaluation device 11, and has a storage function of storing data. Rule data 21 for prediction processing of a metabolic reaction process and reference information for toxicity evaluation 23 are stored in the DB 16. The DB 16 is connected to the toxicity evaluation device 11, for example, through a network such as a LAN. Of course, the DB 16 may be built in the toxicity evaluation device 11.

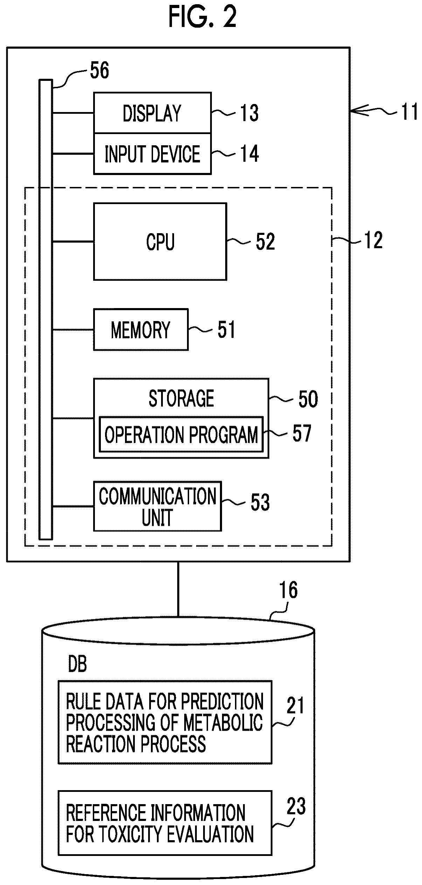

In FIG. 2, the computer constituting the toxicity evaluation device 11 includes a body part 12, a display 13, and an input device 14. These units are connected to each other through a bus line 56. The body part 12 is provided with a storage 50, a memory 51, a central processing unit (CPU) 52, and a communication unit 53.

The storage 50 is a hard disk drive that is built in the computer constituting the toxicity evaluation device 11 or connected to the computer through a cable or a network. Alternatively, the storage 50 is a disk array in which a plurality of hard disk drives are mounted. The storage 50 stores a control program such as an operating system, various application programs including an operation program 57, various types of data associated with these programs, and the like. It should be noted that a solid state drive may be used instead of the hard disk drive.

The memory 51 is a work memory used by the CPU 52 to execute processes. The CPU 52 integrally controls the units of the computer by loading the programs stored in the storage 50 into the memory 51 and executing processing compliant with the programs. The operation program 57 is an application program for causing the computer to function as the toxicity evaluation device 11, and is an example of an "operation program of a toxicity evaluation device" according to the technology of the present disclosure.

The communication unit 53 is a network interface that controls transmission of various types of information via the network. For example, communication with the DB 16 is performed through the communication unit 53. The display 13 displays various screens. The various screens include a screen on which the final processing result 26 is displayed. The toxicity evaluation device 11 receives input of an operation instruction from the input device 14 through various screens.

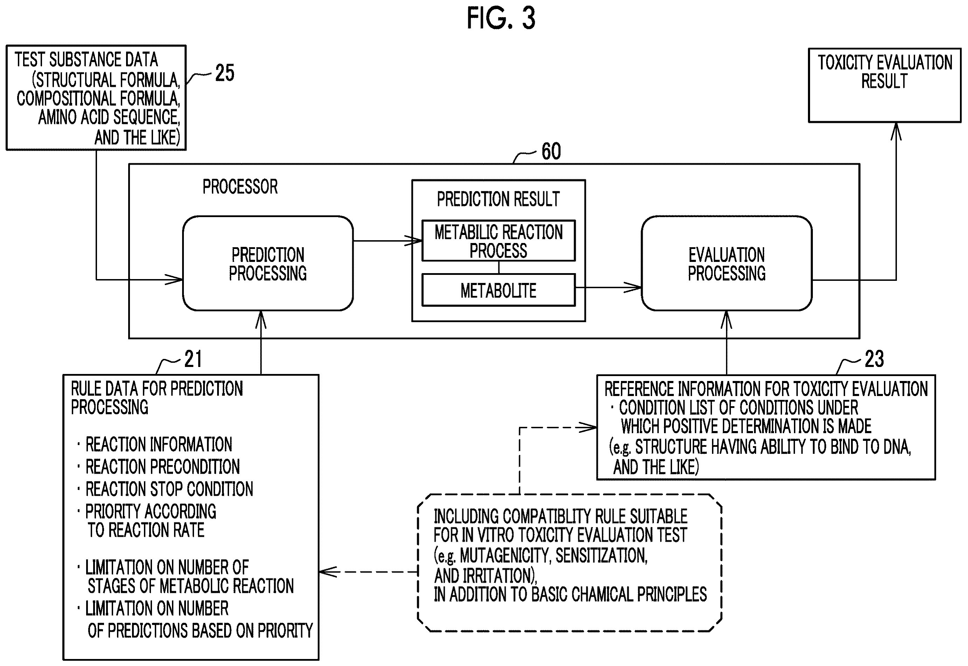

As shown in FIG. 3, in a case where the CPU 52 executes the operation program 57, the CPU 52 functions as a processor 60 that executes the prediction processing and the toxicity evaluation processing. The processor 60 is an example of a processor according to the technology of the present disclosure. The processor 60 executes data acquisition processing, prediction processing, and evaluation processing.

The data acquisition processing is processing of acquiring the test substance data 25. The test substance is any compound to be evaluated for toxicity, and examples thereof include sugars, peptides, proteins, nucleic acids, aromatic compounds, aliphatic compounds, organometallic compounds, inorganic compounds, and the like. The test substance data 25 is data generally used as compound data. The test substance data 25 is, for example, data selected from a structural formula of the test substance, a compositional formula of the test substance, an amino acid sequence, and the like of the test substance.

The user operates the toxicity evaluation device 11 to select one of a plurality of compound data prepared in advance as the test substance data 25, or to input a structural formula or the like of a compound and select the structural formula or the like as the test substance data 25. The processor 60 acquires the data selected by the user as the test substance data 25. In a case where the test substance data 25 is input in a form of a structural formula, a compositional formula, an amino acid sequence, or the like, the processor 60 quantifies the test substance data 25 according to a preset transformation rule.

The processor 60 acquires the test substance data 25, and then executes prediction processing and evaluation processing based on a start instruction of processing by the user. The prediction processing is a processing of predicting a metabolic reaction process of the test substance and a metabolite generated in the metabolic reaction process in a case where the test substance is subjected to an in vitro toxicity evaluation test, and outputting the metabolic reaction process and the metabolite as a prediction result. The processor 60 predicts the metabolic reaction process based on the test substance data 25 and the rule data 21. The rule data 21 is data specifying a rule in which a metabolic reaction proceeds. In the following, the metabolic reaction may be simply referred to as a reaction.

The rule data 21 includes a compatibility rule suitable for an in vitro toxicity evaluation test in addition to a rule reflecting the basic principle of chemistry.

In one embodiment, the in vitro toxicity evaluation test is an evaluation test for mutagenicity, sensitization, or irritation. The evaluation test for mutagenicity, sensitization, or irritation is an in vitro toxicity evaluation test that is generally used for toxicity evaluation of chemical substances. Examples of the evaluation test for mutagenicity include a bacterial reverse mutation test (also referred to as the Ames test), a mouse lymphoma TK assay, a micronucleus test, a chromosomal aberration test, and the like. Examples of the evaluation test of sensitization include a keratinocyte cell line reporter assay, a human cell line activation test, an amino acid derivative reactivity assay (ADRA), a direct peptide reactivity assay (DPRA), and the like. Examples of the evaluation test of irritation include a skin irritation test and an eye irritation test using cultured cells.

The "compatibility rule suitable for an in vitro toxicity evaluation test" is a rule for allowing progression of metabolic reaction prediction to be compatible with an in vitro toxicity evaluation test, based on an evaluation result obtained in an actual in vitro toxicity evaluation test. Since the toxicity evaluation of the in silico method by the toxicity evaluation device 11 is performed theoretically, the evaluation result may deviate from the test result of the toxicity evaluation of the actual in vitro toxicity evaluation test. One of the causes is considered to lie in the metabolic reaction prediction. For example, in a case where the metabolic reaction prediction is performed only based on the theoretical rule without using the compatibility rule, there are cases where predictions that cannot occur in an actual metabolic reaction, or the like are repeatedly made. The compatibility rule is a rule for making the evaluation result by the in silico method closer to the test result of the in vitro toxicity evaluation test. The compatibility rule is created, for example, based on findings inductively extracted from the test results of the in vitro toxicity evaluation test.

The rule data 21 includes, as an example, at least one condition of reaction information, a reaction precondition, a reaction stop condition, a priority according to a reaction rate, a limitation on the number of stages of a metabolic reaction, or a limitation on the number of predictions based on the priority. In addition, as an example, at least one selected from the group consisting of a reaction precondition, a reaction stop condition, and a priority includes a compatibility rule.

The reaction information is information related to a chemical reaction that occurs with respect to the chemical structure of a certain compound. That is, the reaction information is information indicating that a chemical reaction R1 occurs with respect to the chemical structure S1. The reaction precondition is a condition for defining a premise that a metabolic reaction occurs in the in vitro toxicity evaluation test. The reaction stop condition defines a condition under which the metabolic reaction is stopped in the in vitro toxicity evaluation test.

As an example, one reaction precondition is attached to one piece of reaction information. In some cases, one reaction stop condition is attached to one piece of reaction information in addition to one reaction precondition. In a case where the reaction precondition is attached and the reaction stop condition is not attached, it can be predicted that the reaction defined in the corresponding reaction information proceeds in a case where the reaction precondition is satisfied. In a case where the reaction stop condition is added in addition to the reaction precondition, it can be predicted that the reaction defined in the corresponding reaction information is stopped in a case where the reaction stop condition is satisfied.

The priority is an evaluation value given for each type of metabolic reaction to preferentially handle a metabolic reaction that is likely to occur in the in vitro toxicity evaluation test. The priority is, as an example, a priority determined based on at least one reaction rate selected from the group consisting of a reaction rate by a metabolic enzyme used in an in vitro toxicity evaluation test and a reaction rate by a non-enzymatic reaction.

The limitation on the number of predictions based on the limitation on the number of stages of the metabolic reaction and the priority is setting information for limiting the prediction of the metabolic reaction. The rule data 21 will be described below.

The evaluation processing is a processing of evaluating the toxicity of at least one compound selected from the group consisting of a test substance and a metabolite and acquiring an evaluation result. The evaluation processing is executed after the prediction processing. In the evaluation processing, the processor 60 determines whether or not at least one compound selected from the group consisting of the test substance and the metabolite corresponds to a compound exhibiting toxicity or has a chemical structure exhibiting toxicity, based on the reference information for toxicity evaluation 23. The compound to be evaluated for toxicity may be both a test substance and a metabolite, or may be either a test substance or a metabolite. Even in a case where a plurality of metabolites are generated in the metabolic reaction process, all the metabolites may be subject to toxicity evaluation, or only a part of the metabolites may be subject to toxicity evaluation. In the evaluation processing, in a case where the toxicity of both the test substance and the metabolite are evaluated, the toxicity of both the test substance itself and the metabolite obtained by chemically transforming the test substance can be evaluated.

The reference information for toxicity evaluation 23 is information that is referred to in the evaluation of toxicity. Examples of the reference information for toxicity evaluation 23 include a condition list of conditions under which a positive determination is made. The conditions under which a positive determination is made include, for example, a condition such as whether or not the structure that binds to DNA is included. The conditions included in the condition list are set based on toxic compound information based on documents such as Chem. Rev. 2011, 111, 2507-2536, software such as QSAR Toolbox, and the like.

The reference information for toxicity evaluation 23 also includes information suitable for an in vitro toxicity evaluation test, in addition to the information reflecting the basic principles of chemistry. The in vitro toxicity evaluation test is a test for evaluating the toxicity of a test substance under artificially configured conditions such as in a test tube. In a case where the reference information for toxicity evaluation 23 includes information suitable for the in vitro toxicity evaluation test, for example, in a case where the transformation to a compound determined to be toxic in a specific in vitro toxicity evaluation test proceeds, the compound to be evaluated is determined to be positive. The condition such as whether or not the structure that binds to DNA is included is an example of information suitable for an in vitro toxicity evaluation test. In addition, examples of the information suitable for the in vitro toxicity evaluation test include information on a structure that binds to a protein, a structure that is intercalated between base pairs of a double-stranded DNA, a structure that destroys a cell membrane, and the like.

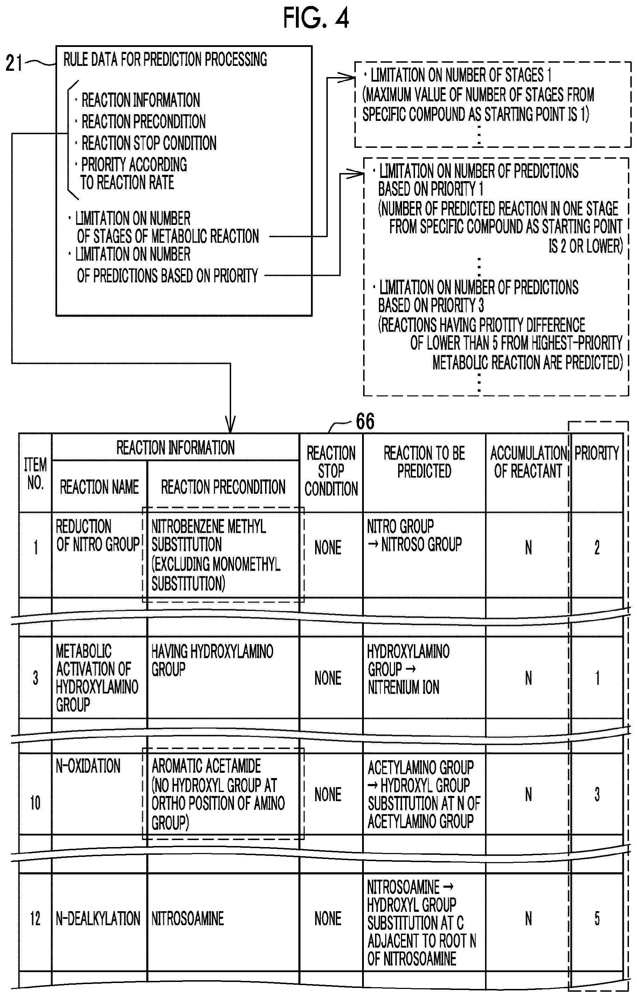

The rule data 21 will be specifically described with reference to FIG. 4. As shown in FIG. 4 as an example, the reaction information, the reaction precondition, the reaction stop condition, and the priority of the rule data 21 are organized in a format of table data 66. In the table data 66, the reaction information, the reaction precondition, the reaction stop condition, and the priority are recorded for each metabolic reaction as an example.

In addition, the table data 66 of the present example includes each item of the predicted reaction and the accumulation of the reactant. The predicted reaction specifically indicates a chemical change of a compound in the reaction. The accumulation of the reactant indicates whether or not the compound existing before the metabolic reaction remains accumulated after the reaction. "N" means that there is no accumulation, and "Y" means that there is accumulation, although not shown.

In the table data 66 of the present example, for example, the metabolic reaction of item no. 1 is "reduction of nitro group", and the reaction precondition is "nitrobenzene methyl substitution (excluding monomethyl substitution)". The reaction stop condition is "none". The predicted reaction is "nitro group → nitroso group", the accumulation of the reactant is "N", and the priority is given as "2".

In the table data 66, a portion indicated by a broken line is an example of the compatibility rule. Since the priority is an evaluation value indicating the likelihood of the metabolic reaction, it is possible to make the prediction of the metabolic reaction closer to the test result of the in vitro toxicity evaluation test by handling the metabolic reaction according to the priority in the prediction processing. In addition, the reaction preconditions of item no. 1 and item no. 10 are also rules inductively extracted from the test results of the in vitro toxicity evaluation test, and it is possible to make the prediction of the metabolic reaction closer to the test results of the in vitro toxicity evaluation test by applying this reaction precondition in the prediction processing.

In the prediction processing, the processor 60 predicts the metabolic reaction of the test substance while comparing the acquired test substance data 25 with the table data 66. Then, in a case where there is a metabolite generated by the metabolic reaction, the metabolic reaction of the metabolite is predicted while comparing the data of the metabolite with the table data 66. The processor 60 repeats such reaction prediction processing in the prediction processing.

The reaction prediction processing is a processing of predicting a metabolic reaction that is repeatedly performed stepwise starting from the test substance and a metabolite generated by the metabolic reaction at each stage. The expression "repeatedly performed stepwise" in the reaction prediction processing refers to a situation in which a plurality of metabolic reactions occur successively, such that one compound undergoes a metabolic reaction, and a metabolite generated by the metabolic reaction further undergoes a metabolic reaction to generate another metabolite. One stage means a stage in which one or a plurality of reactions occur starting from one compound.

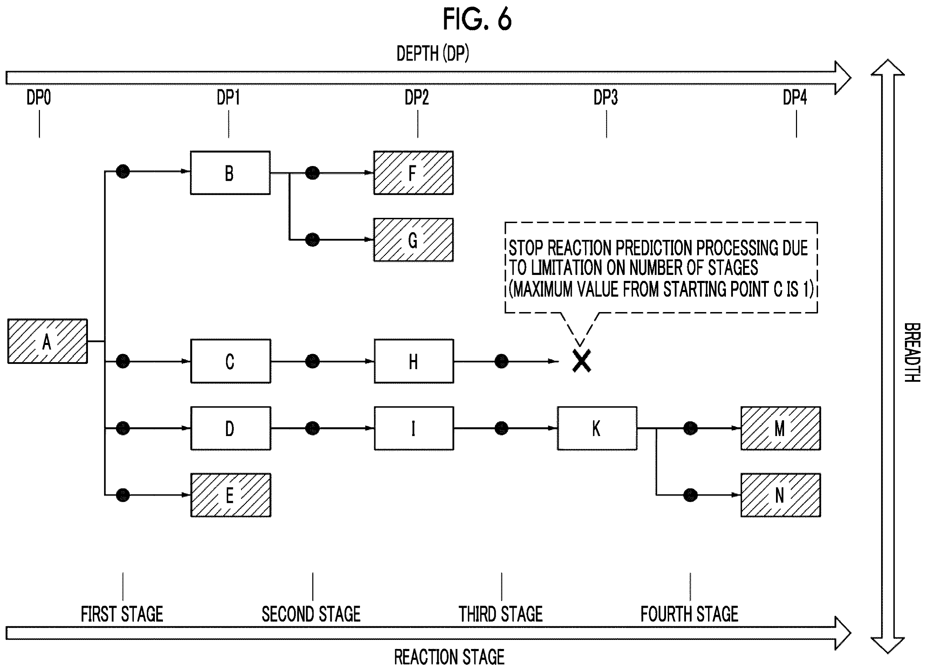

The limitation on the number of stages of the metabolic reaction and the limitation on the number of predictions based on the priority are setting information for suppressing the prediction of a reaction that is not suitable for the in vitro toxicity evaluation test, and are an example of a compatibility rule. The limitation on the number of stages of the metabolic reaction means limiting the prediction of the metabolic reaction depending on the number of stages of the metabolic reaction. As shown in FIG. 6 as an example, the limitation on the number of stages 1 is defined in a form of "maximum value of the number of stages from a specific compound as a starting point = 1". In a case where there is such a limitation on the number of stages 1, the processor 60 predicts the first-stage metabolic reaction from a specific compound as a starting point, but stops the prediction of the metabolic reactions of the second stage and subsequent stages.

On the other hand, the limitation on the number of predictions based on the priority means limiting the number of predictions of the metabolic reaction according to the priority given to the metabolic reaction. By limiting the number of predictions based on the priority, the metabolic reaction to be predicted is narrowed down to a metabolic reaction having a relatively high priority.

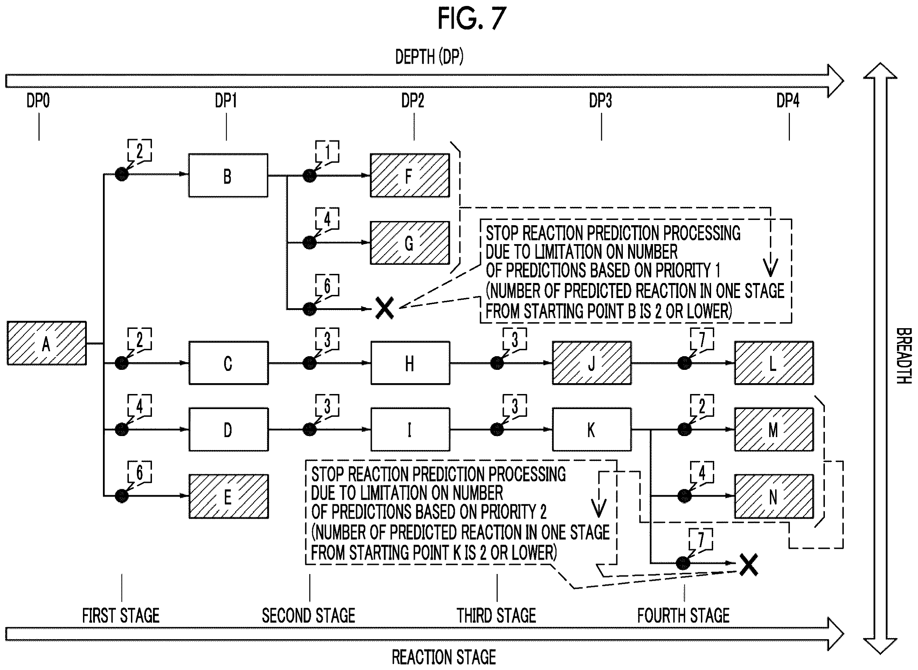

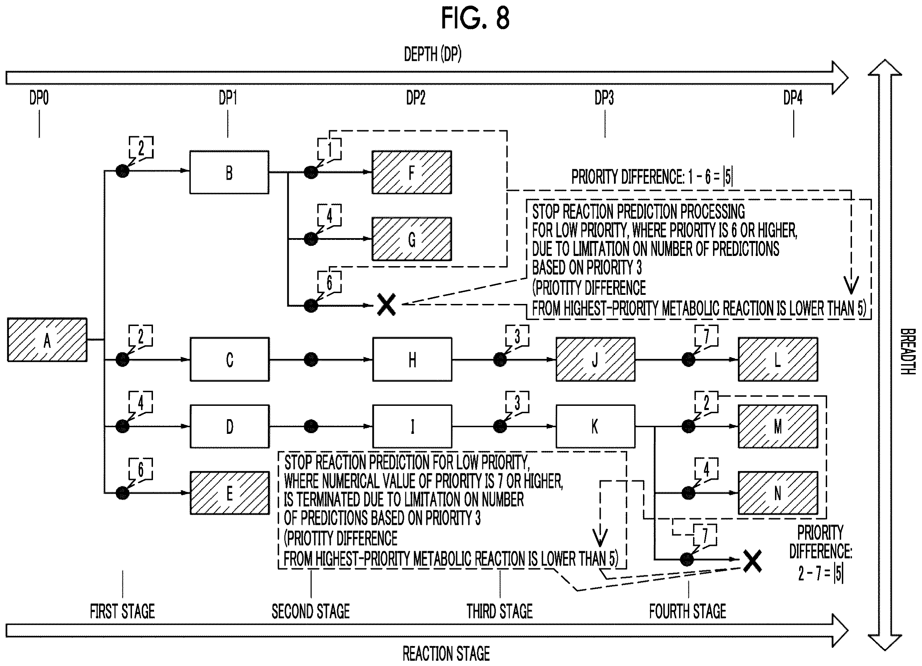

As shown in FIG. 7 as an example, the limitation on the number of prediction 1 or 2 based on the priority is defined in a form such as "the number of predictions of one-stage reaction from a specific compound as a starting point is 2 or less". One compound may undergo not only one but a plurality of metabolic reactions. In a case where there is such a limitation on the number of prediction, the processor 60 stops the prediction for the third reaction even in a case where there are three or more predicted reactions in one step from a specific compound as a starting point. As a result, the number of predictions of the reaction is limited to two according to the priority. In addition, the limitation on the number of predictions 3 based on another priority shown in FIG. 8 is defined in a form of "predicting a reaction in which a difference in priority from the metabolic reaction having the highest priority is less than 5". This is also a definition for limiting the prediction range such that the difference in priority is less than 5 on the basis of the reaction with the highest priority among the plurality of reactions in a case where a plurality of reactions can be predicted in one step. In a case where there is such a limitation on the number of predictions 3, the processor 60 stops the prediction for the reaction in which the difference in priority from the reaction with the highest priority is 5 or more.

In addition, it is also possible to set such that the limitation on the number of predictions based on the priority is invalidated for the first-stage metabolic reaction from the test substance as a starting point and validated for the second-stage and subsequent metabolic reactions. In this case, all metabolic reactions that occur for the first-stage metabolic reaction from the test substance as a starting point are predicted.

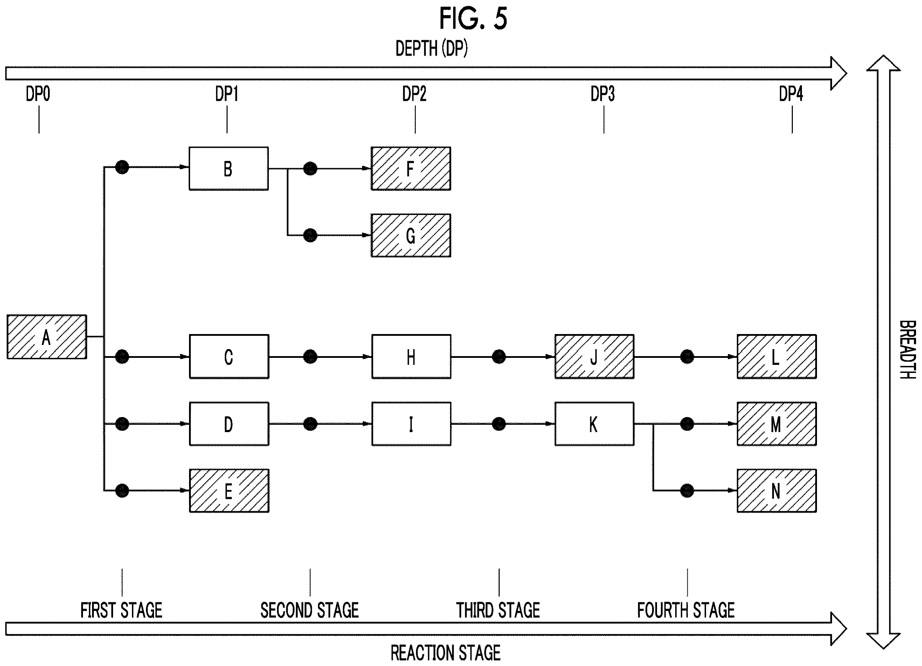

The prediction processing will be described in more detail with reference to FIGS. 5 to 8. FIG. 5 conceptually shows a pathway in which the test substance generates a plurality of metabolites through a metabolic reaction that is repeatedly performed stepwise in the prediction processing. In FIG. 5, the test substance is shown as a compound A, and the metabolites are shown as compounds B, C, E, F, and the like. In addition, the metabolic reaction is represented by a line connecting compounds and a black circle.

In the metabolic reaction process shown in FIG. 5, in the first-stage metabolic reaction, the compounds B, C, D, and E are generated using the compound A as a reactant. In the second-stage metabolic reaction, the compounds F and G are generated using the compound B as a reactant, the compound H is generated using the compound C as a reactant, and the compound I is generated using the compound D as a reactant. In the third-stage metabolic reaction, the compound J is generated using the compound H as a reactant, and the compound K is generated using the compound I as a reactant. In the fourth-stage metabolic reaction, the compound L is generated using the compound J as a reactant, and the compounds M and N are generated using the compound K as a reactant.

Here, in the metabolic reaction process shown in FIG. 5, a direction in which the stage of the metabolic reaction proceeds is indicated by the concept of depth. The depth DP increases by one each time one stage of a metabolic reaction is passed. In the present example, the depth DP of the compound A as a test substance is defined as "DP0". Then, with respect to the compound A, the depth DP of the compounds B to E generated by the first-stage metabolic reaction is defined as "DP1", and the depth DP of the compounds F to I generated by the second-stage metabolic reaction next to the first-stage metabolic reaction is defined as "DP2". The same applies to the third and subsequent stages.

In addition, in a case where a plurality of metabolic reactions occur in one stage, the number of metabolic reactions at the same stage is represented by the concept of breadth. In the first-stage metabolic reaction, four compounds of the compounds B to E are generated, and in the two metabolic reactions occurring in the compound B, two compounds of the compounds F and G are generated. The breadth increases as the number of metabolic reactions occurring in one stage increases.

In the prediction processing, the processor 60 predicts what type of metabolic reaction occurs starting from the test substance, and what type of metabolite is generated by the metabolic reaction, while referring to the rule data 21.

As described above, in the present example, the rule data 21 is defined for each type of metabolic reaction. The processor 60 executes the reaction prediction processing for each stage and each metabolic reaction based on the rule data 21. For example, with reference to the metabolic reaction process in FIG. 5, the processor 60 executes, based on the rule data 21, the reaction prediction processing for each stage and each metabolic reaction (for example, for each of the reaction from the compound A to the compound B, the reaction from the compound A to the compound C, the reaction from the compound A to the compound D, and the reaction from the compound A to the compound E). Since the metabolic reaction occurs successively, the prediction results of the metabolic reaction process and the metabolite have a form like a dendrogram shown in FIG. 5.

In FIG. 5, the compounds (compounds A, E, F, G, J, L, M, and N) indicated by hatching with diagonal lines are compounds to be evaluated for toxicity in the evaluation processing. In this way, not all the metabolites in the metabolic reaction process are necessarily subject to toxicity determination. As an example, whether or not the substance is subject to toxicity evaluation is determined according to the presence or absence of "accumulation of reactant" as shown in the table data 66 of FIG. 4. That is, in the metabolic reaction process shown in FIG. 5, among the metabolites produced from the test substance A, the compounds E, F, G, J, L, M, and N are "Y: present" in the "accumulation of reactant", and are compounds accumulated in the system. On the other hand, the compounds B, C, D, H, I, and K are "N: absent" in the "accumulation of reactant", and are transformed into the next metabolite and are not accumulated in the system. Therefore, the compound that accumulates in the system and the compound A that is a test substance are subjects to toxicity evaluation.

In the reaction prediction processing, the toxicity evaluation device 11 can limit a maximum value of the number of stages of a metabolic reaction that is repeatedly performed stepwise starting from the test substance or the metabolite based on a structure of the test substance or the metabolite. This limitation is performed based on the limitation on the number of stages defined in the rule data 21. As a limitation on the number of stages, it is assumed that the maximum value of the number of stages of the metabolic reaction from the compound C as a starting point is 1. In this case, as shown in FIG. 6, the processor 60 performs the reaction prediction processing for the one-stage metabolic reaction from the compound C as a starting point, and predicts the compound H as a metabolite. However, since the maximum value of the number of stages from the compound C as a starting point is 1, the processor 60 stops the reaction prediction processing for the metabolic reaction occurring for the compound H.

In this way, by performing limitation on the number of stages, unnecessary prediction processing for the metabolic reaction and the metabolite that do not affect the toxicity evaluation result can be omitted. The limitation on the number of stages is a limitation in the depth direction in the metabolic reaction process.

FIGS. 7 and 8 show an example in which limitation on the number of predictions is performed for the reaction prediction processing. In the reaction prediction processing, in a case where a plurality of predictions of a metabolic reaction occurring in one stage can be made with a test substance or a metabolite as a starting point, the toxicity evaluation device 11 can limit the number of predictions of the metabolic reaction in the stage.

The limitation on the number of predictions is performed based on the priority. In a case where the number of predictions based on the priority, a reaction that is unlikely to occur in the in vitro toxicity evaluation test, that is, a reaction with a low priority is limited, and the unnecessary prediction of an metabolite can be prevented.

As shown in the table data 66 of FIG. 4, the priority is set for each metabolic reaction. In FIG. 7, a number within a broken line frame attached to a black circle indicating each metabolic reaction indicates a priority set for each metabolic reaction. In the example shown in FIG. 7, the limitation 1 and the limitation 2 are set as the limitation on the number of predictions based on the priority. Limitation 1 is a condition that "the number of predicted metabolic reactions in one stage from the compound B as a starting point is 2 or less", and Limitation 2 is a condition that "the number of predicted reactions in one stage from the compound K as a starting point is 2 or less".

In FIG. 7, it is assumed that three or more types of metabolic reactions occur in one stage from the compound B as a starting point. The processor 60 executes the reaction prediction processing for the metabolic reactions having relatively high priorities (priorities "1" and "4"), and predicts the compound F and the compound G as the generated metabolites. On the other hand, for the metabolic reaction having a relatively low priority (priority "6"), the reaction prediction processing is stopped due to the limitation 1 that the number of predictions of the metabolic reaction is 2 or less. Similarly, it is assumed that three or more types of metabolic reactions occur in one stage from the compound K as a starting point. The processor 60 executes the reaction prediction processing for two metabolic reactions (priorities "2" and "4") having relatively high priorities, and predicts the compound M and the compound N as the generated metabolic products. On the other hand, for the metabolic reaction having a relatively low priority (priority "7"), the reaction prediction processing is stopped due to the limitation 1 that the number of predictions of the metabolic reaction is 2 or less.

In addition, in a case where a stage of a metabolic reaction occurring in the test substance is defined as a first stage and a stage of a metabolic reaction occurring in the metabolite generated by the metabolic reaction in the first stage is defined as a second stage, the limitation on the number of predictions based on the priority is performed for the metabolic reactions in the second stage and subsequent stages. In the in vivo reaction, the environment in which the enzyme approaches may differ between a case where the enzyme approaches the compound to cause a first-stage structural transformation and a case of causing a second or higher stage structural transformation. For example, the first-stage metabolic reaction easily occurs in a case where an enzyme approaches a compound, but the second-stage and subsequent-stage metabolic reactions may not easily occur even in a case where an enzyme approaches a compound. Therefore, in a case where the number of predictions based on the priority is limited for the second-stage and subsequent-stage metabolic reactions, the prediction of a reaction that is unlikely to occur in the in vitro toxicity evaluation test is limited, and the unnecessary prediction of a metabolite can be prevented. On the other hand, since the progression of the first-stage metabolic reaction is not likely to be inhibited, it is considered that, in a case where for the first-stage metabolic reaction, the limitation on the number of predictions based on the priority is not performed, a suitable result for the in vitro toxicity evaluation test is obtained.

For example, as shown in FIG. 7, the reaction prediction processing is performed for four metabolic reactions in the first stage from the test substance A as a starting point, and the Limitation that the number of predictions is set to 2 or less is invalidated. On the other hand, for the second-stage and subsequent-stage metabolic reactions from the compounds B, C, D, and E as starting points, the Limitation that the number of predictions in one stage is 2 or less is validated.

In one embodiment, the limitation on the number of predictions is performed by limiting the prediction of the metabolic reaction occurring in one stage to the metabolic reaction in which a difference in priority with the metabolic reaction having the highest priority among a plurality of metabolic reactions that do not correspond to the reaction stop condition and correspond to the reaction precondition is within a preset range. As an example, the preset range is less than 5, may be less than 4, or may be less than 3.

In FIG. 8, the metabolic reaction is limited based on the difference in priority is performed by the limitation on the number of predictions 3 based on the priority. That is, the prediction of the metabolic reaction generated from the compound B as starting point is limited to the metabolic reaction in which the difference in priority from the metabolic reaction having the highest priority among the plurality of metabolic reactions that do not correspond to the reaction stop condition and correspond to the reaction precondition is less than 5. In FIG. 8, as shown in FIG. 7, the priority for each metabolic reaction is indicated by the number within a broken line frame. In the example of FIG. 8, among a plurality of metabolic reactions in one stage from the compound B as a starting point, the metabolic reaction having the highest priority is a metabolic reaction from the compound B to the compound F, and the priority of the metabolic reaction having the highest priority is "1". Therefore, since the difference from the highest priority is 5 or more for the low priority metabolic reaction in which the numerical value of the priority descending from the highest priority is "6" or more, the reaction prediction processing is stopped and the prediction is not performed. The same limitation 3 is set for the compound K. Among a plurality of metabolic reactions generated from the compound K as a starting point, the numerical value of the priority of the metabolic reaction having the highest priority (the metabolic reaction from the compound K to the compound M) is "2". Therefore, the reaction prediction processing of the metabolic reaction having low priority in which the numerical value of the priority is "7" or more is stopped, and the prediction is not performed.

As in the limitation on the number of predictions based on such a priority, the limitation on the number of predictions of the metabolic reaction occurring in one stage is a limitation in the breadth direction in the metabolic reaction process. In addition, the prediction processing having a relatively low priority is considered to be a metabolic reaction having a relatively low influence degree on the toxicity evaluation result. In a case where limitation on the number of predictions based on the priority is performed, the prediction processing of the metabolic reaction having a relatively low influence degree on the toxicity evaluation result can be omitted.

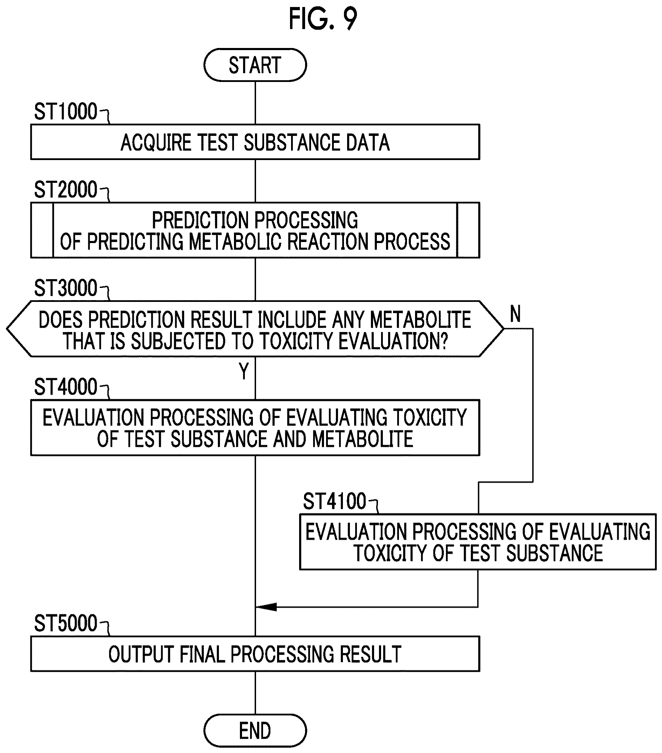

The operation of the configuration described above will be described with reference to the flowcharts of FIGS. 9 to 11. FIG. 9 is a flowchart showing an example of an overall processing procedure of the toxicity evaluation device 11. First, the processor 60 acquires the test substance data 25 selected by the user (Step ST1000). Subsequently, the processor 60 executes prediction processing of predicting the metabolic reaction process (Step ST2000). The processor 60 determines whether or not there is a metabolite to be evaluated for toxicity in the prediction result of Step ST2000 (Step ST3000). In a case of a positive determination in Step ST3000, the processor 60 proceeds to Step ST4000 and executes the toxicity evaluation processing of evaluating the toxicities of the test substance and the metabolite. On the other hand, in a case of a negative determination in Step ST3000, the processor 60 proceeds to Step ST4100 and executes the evaluation processing of evaluating the toxicity of only the test substance. Subsequently, the processor 60 outputs the final processing result including the evaluation results obtained in Step ST4000 and Step ST4100 (Step ST5000). The final processing result may include data on the metabolic reaction process and the metabolite, in addition to the toxicity evaluation results of the test substance and the metabolite.

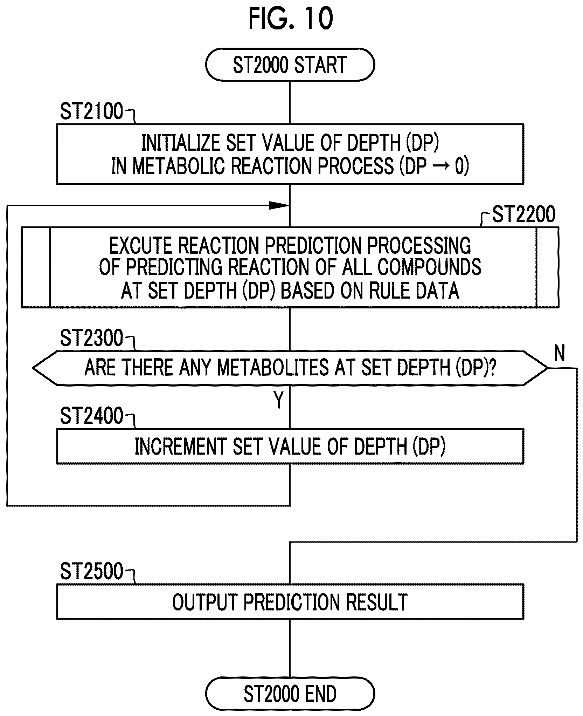

FIG. 10 is a flowchart showing an example of a processing procedure of the prediction processing (Step ST2000) shown in FIG. 9. In the prediction processing, a metabolic reaction process as shown in FIG. 5 or the like is predicted. The prediction of the metabolic reaction process is performed from the test substance as a starting point at the depth DP0. Therefore, first, the processor 60 initializes the set value of the depth DP of the metabolic reaction process and sets the depth DP to 0 (Step ST2100). Subsequently, the reaction prediction processing of predicting the reaction of the test substance based on the rule data 21 is executed (Step ST2200). Whether or not there are any metabolites is determined at the depth DP0 (Step ST2300), and in a case of a positive determination, the set value of the depth DP is incremented (Step ST2400), and the depth DP is set to 1. Next, the reaction prediction processing of predicting the metabolic reaction based on the rule data 21 is executed for all the compounds (metabolites) in DP1 (Step ST2200). Hereinafter, Steps ST2300, ST2400, and ST2200 are repeated in the same manner. In Step ST2300, in a case where there is no metabolite at the set depth DP, the reaction prediction processing of the metabolic reaction process is terminated, and the prediction results of the metabolic reaction process and the metabolite are output (Step ST2500). For example, the prediction result is temporarily stored in the storage 50 or the like in preparation for the output of the final processing result shown in FIG. 9. As shown in FIG. 9, after the prediction processing in Step ST2000 is terminated, the processor proceeds to the toxicity evaluation processing.

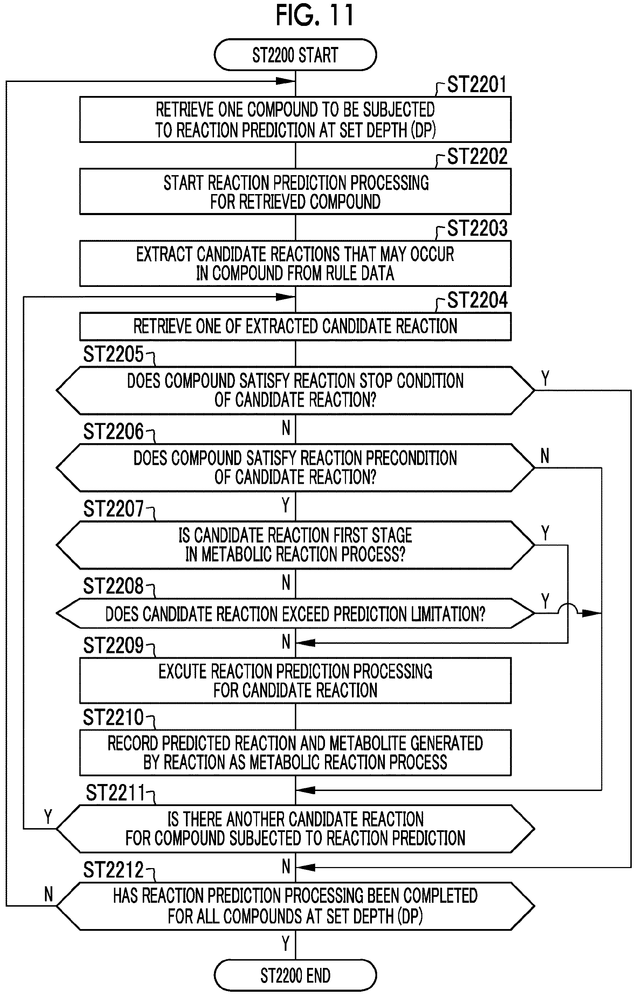

FIG. 11 is a flowchart showing an example of a processing procedure of the reaction prediction processing (Step ST2200) shown in FIG. 10. In Step ST2201, the processor 60 reads out one compound that is subject to the reaction prediction at the set depth DP. In a case where the depth DP is the initial state (depth DP0), in Step ST2201, the processor 60 first reads out the test substance data 25 as the data of the compound in a case where the set depth DP is DP0. On the other hand, in a case where the depth DP is DP1 or later, the data of the compound of the metabolite is read out. Then, the reaction prediction processing of the compound, which is the read-out compound, is started (Step ST2202).

Subsequently, in Step ST2203, the processor 60 compares the data of the compound with the rule data 21 to extract candidates for the reaction that may occur in the compound. The processor 60 reads out one extracted candidate for the reaction (Step ST2204) and determines whether or not the compound corresponds to the reaction stop condition of the reaction as a candidate (Step ST2205).

In a case of a positive determination in Step ST2205 (Y in Step ST2205), that is, in a case where the compound that is subject to the reaction corresponds to the reaction stop condition of the reaction as a candidate, the processor 60 terminates the reaction prediction processing for the compound as a candidate. Then, the processor 60 proceeds to Step ST2212. In a case where the reaction prediction processing has not been terminated for all the compounds at the set depth DP in Step ST2212 (N in Step ST2212), the processer returns to Step ST2201, read out one compound that is subject to a reaction prediction, and proceeds to Step ST2202 again.

On the other hand, in a case of a negative determination in Step ST2205 (N in Step ST2205), that is, in a case where the compound that is subject to the reaction prediction does not correspond to the reaction stop condition of the reaction as the candidate, the processor 60 proceeds to Step ST2206. In step ST2206, the processor 60 determines whether or not the compound that is subject to the reaction prediction corresponds to the reaction precondition of the metabolic reaction as the candidate. In a case of a negative determination in Step ST2206 (N in Step ST2206), the processor 60 terminates the reaction prediction processing for the reaction as the candidate, proceeds to Step ST2211, and in a case where there is another reaction as the candidate, returns to Step ST2204.

On the other hand, in a case of a positive determination in Step ST2206 (Y in Step ST2206), the processor 60 determines whether or not the reaction as the candidate is the first stage of the metabolic reaction process (Step ST2207).

In a case where the compound that is subject to the reaction prediction is a test substance which is a compound having the depth DP0, the reaction generated by the test substance is a first-stage metabolic reaction. Therefore, in a case where the subject to the reaction prediction is a test substance, a positive determination (Y in Step ST2207) is made in Step ST2207. The processor 60 proceeds to ST2209 without limitation on the number of predictions. In Step ST2209, the processor 60 executes the reaction prediction processing for the reaction as a candidate. Then, in Step ST2210, the predicted metabolic reaction and the metabolite generated by the metabolic reaction are recorded as the metabolic reaction process.

On the other hand, in a case of a negative determination in Step ST2207 (N in Step ST2207), for example, in a case where the metabolic reaction as the candidate is a metabolic reaction of a metabolite other than the test substance, the processor 60 proceeds to Step ST2208. In Step ST2208, the processor 60 determines whether or not the metabolic reaction as the candidate corresponds to the prediction limitation. For example, in a case where there is a limitation on the number of stages as shown in FIG. 6, the processor 60 determines whether or not the number of stages of the metabolic reaction as a candidate is within the limitation. In addition, for example, in a case where there is a limitation on the number of predictions based on the priority as shown in FIGS. 7 and 8, the processor 60 determines whether or not the metabolic reaction as the candidate corresponds to the limitation on the number of predictions based on the priority. In a case where the metabolic reaction as the candidate corresponds to the prediction limitation (Y in Step ST2208), the processor 60 stops the reaction prediction processing and proceeds to Step ST2211.

On the other hand, in a case of a negative determination in Step ST2208 (N in Step ST2208), that is, in a case where the metabolic reaction as the candidate does not correspond to the prediction limitation, the processor 60 proceeds to Step ST2209 and executes the reaction prediction processing for the metabolic reaction as the candidate. Then, in Step ST2210, the metabolic reaction and the metabolite are recorded as the metabolic reaction process.

The processing from Step ST2204 to Step ST2210 is repeated until a negative determination (N in Step ST2211) is made in Step ST2211, that is, until it is determined that there is no other reaction as a candidate. In a case of a negative determination in Step ST2211, the processor 60 proceeds to Step ST2212. In Step ST2212, the processor 60 determines whether or not the reaction prediction processing has been terminated for all the compounds at the set depth DP. In a case of the depth DP0, since the compound that is subject to the reaction prediction is only the test substance, in a case where the reaction prediction processing of the test substance is terminated, a positive determination is made in Step ST2212 (Y in Step ST2212). On the other hand, in a case where the depth DP is DP1 or later, a plurality of metabolites may be present. In this case, a negative determination is made in Step ST2212 (N in Step ST2212), and the processor 60 returns to Step ST2201 and repeats the processing after Step ST2201.

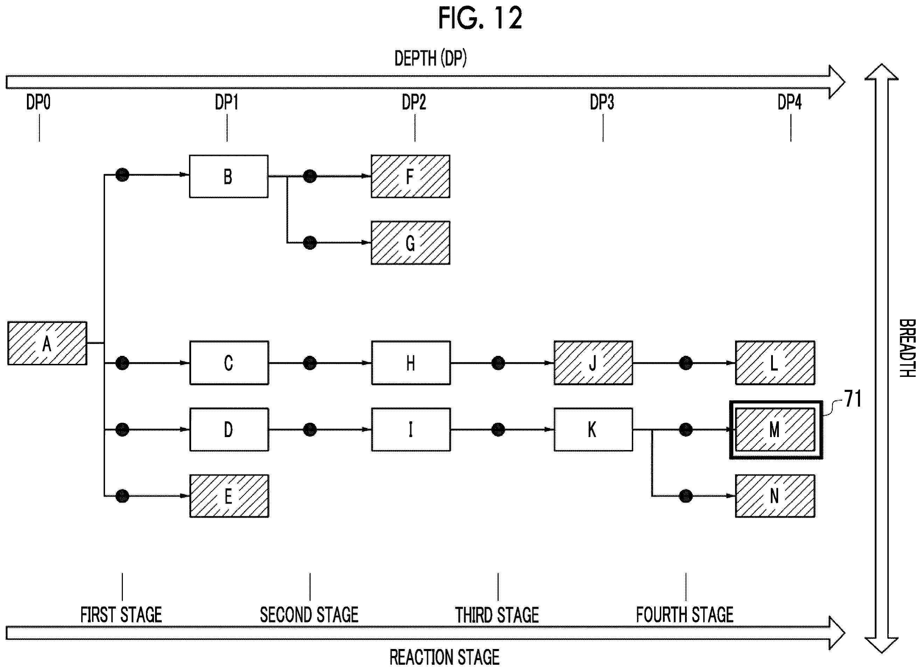

The toxicity evaluation device 11 outputs a final processing result 26 by performing prediction processing and evaluation processing shown in FIGS. 9 to 11 as an example. FIG. 12 is an example of the final processing result 26. In FIG. 12, a metabolic reaction process including a test substance and a metabolite is shown in the form shown in FIG. 5 and the like. Then, the compounds to be evaluated for toxicity are hatched with diagonal lines. With such display, the user can grasp which is a compound to be evaluated for toxicity. In addition, in the metabolic reaction process shown in FIG. 12, for the compound determined to have toxicity, for example, a thick line frame 71 is displayed as in the compound M. With such display, the user can grasp the presence or absence of toxicity of the compound to be evaluated.

As described above, a toxicity evaluation device 11 according to the technology of the present disclosure includes a processor 60, in which the processor 60 is configured to perform data acquisition processing of acquiring test substance data 25 specifying a test substance, prediction processing of predicting a metabolic reaction process of the test substance and a metabolite generated in the metabolic reaction process based on the test substance data 25, evaluation processing of acquiring an evaluation result by evaluating toxicity of at least one compound selected from the group consisting of the test substance and the metabolite, and output processing of outputting a processing result including the evaluation result. In the prediction processing, the processor 60 is configured to predict the metabolic reaction process based on the test substance data 25 and rule data 21 specifying a rule in which a metabolic reaction proceeds, and the rule data 21 includes a compatibility rule suitable for an in vitro toxicity evaluation test. Therefore, it is possible to improve the prediction accuracy even in the toxicity evaluation by the in silico method.

In addition, the test substance data 25 is, for example, data selected from the group consisting of a structural formula of the test substance, a compositional formula of the test substance, and an amino acid sequence of the test substance. The structural formula, the compositional formula, and the amino acid sequence are general data of compounds, and thus are easy to use.

In addition, as an example, the rule data 21 includes at least one condition selected from the group consisting of a reaction precondition that defines a premise under which a metabolic reaction occurs in the in vitro toxicity evaluation test, a reaction stop condition that defines a condition for stopping the metabolic reaction in the in vitro toxicity evaluation test, and a priority for preferentially handling a metabolic reaction that is likely to occur in the in vitro toxicity evaluation test. Then, at least one selected from the group consisting of a reaction precondition, a reaction stop condition, and a priority includes a compatibility rule. As described above, by subdividing and defining the conditions for causing the metabolic reaction, it is possible to expect an effect of easily constructing the logic of the prediction processing for obtaining a result suitable for the toxicity evaluation test in the rule data 21, the operation program, or the like.

In addition, as an example, the priority is a priority given for each type of the metabolic reaction, and the priority includes a priority determined based on at least one reaction rate selected from the group consisting of a reaction rate by a metabolic enzyme used in the in vitro toxicity evaluation test and a reaction rate by a non-enzymatic reaction. By using the priority suitable for such an in vitro toxicity evaluation test, it is possible to make a highly accurate prediction more suitable for the in vitro toxicity evaluation test. In addition, by taking into account at least one selected from the group consisting of the reaction rate by the metabolic enzyme and the reaction rate by the non-enzymatic reaction, it is possible to make a more accurate prediction.

In addition, as an example, the prediction processing includes a reaction prediction processing of predicting a metabolic reaction that is repeatedly performed stepwise starting from the test substance and a metabolite generated by the metabolic reaction at each stage. As a result, it is possible to accurately predict the metabolic reaction occurring successively.

In addition, as an example, the rule data 21 is defined for each type of the metabolic reaction, and the processor 60 is configured to execute the reaction prediction processing, based on the rule data 21, for each of the stages and each of the metabolic reactions. As a result, it is possible to systematically predict the metabolic reaction occurring successively.

In addition, as an example, in the reaction prediction processing, a maximum value of the number of stages of a metabolic reaction that is repeatedly performed stepwise starting from the test substance or the metabolite is limited based on a structure of the test substance or the metabolite. In a case where the number of stages of the metabolic reaction is limited, unnecessary prediction processing can be omitted for a compound that does not affect the toxicity evaluation result even in a case where the number of stages is limited.

In addition, as an example, in the reaction prediction processing, in a case where a plurality of predictions of the metabolic reaction occurring in one of the stages starting from the test substance or the metabolite are possible, limitation on the number of predictions of the metabolic reaction in the stage is possible. In a case where the number of predictions of a metabolic reaction at a certain stage, unnecessary prediction processing such as prediction of a compound that does not affect the toxicity evaluation result can be omitted.

In addition, as an example, the compatibility rule includes a priority for preferentially handling a metabolic reaction that is likely to occur in the in vitro toxicity evaluation test, and the limitation on the number of predictions is performed based on the priority. In a case where the number of predictions is limited based on the priority, a reaction that is unlikely to occur in the in vitro toxicity evaluation test, that is, a reaction with a low priority is limited, and unnecessary prediction processing can be omitted.

In addition, as an example, in a case where a stage of a metabolic reaction occurring in the test substance is defined as a first stage and a stage of a metabolic reaction occurring in the metabolite generated by the metabolic reaction in the first stage is defined as a second stage, the limitation on the number of predictions based on the priority is performed for the metabolic reactions in the second stage and subsequent stages. In a case where the number of predictions based on the priority is limited for the metabolic reaction in the second stage and subsequent stages, the prediction of a reaction that is unlikely to occur in the in vitro toxicity evaluation test is limited, and unnecessary prediction processing can be omitted. On the other hand, it is considered that, for the first-stage metabolic reaction, in a case where limitation on the number of predictions based on the priority is not performed, a more suitable result is obtained by the in vitro toxicity evaluation test.

In addition, as an example, the compatibility rule includes a reaction precondition that defines a premise under which a metabolic reaction occurs in the in vitro toxicity evaluation test, a reaction stop condition that defines a condition for stopping the metabolic reaction in the in vitro toxicity evaluation test, and a priority for preferentially handling a metabolic reaction that is likely to occur in the in vitro toxicity evaluation test, and the limitation on the number of predictions is performed by limiting a prediction of the metabolic reaction occurring in one of the stages to a metabolic reaction in which a difference in priority from a metabolic reaction having the highest priority among a plurality of metabolic reactions not satisfying the reaction stop condition and satisfying the reaction precondition is within a preset range. After determining whether or not the reaction stop condition and the reaction precondition are satisfied, in a case where the number of predictions is limited according to the difference in priority from the metabolic reaction having the highest priority, unnecessary prediction processing can be efficiently omitted for each compound which is a reactant. As an example, the preset range is less than 5.

In addition, as an example, as shown in Step ST2205 of FIG. 11, in a case where one metabolic reaction as a candidate for one compound corresponds to the reaction stop condition, the reaction prediction processing for other metabolic reactions for the compound is stopped. This is because, in a case where one metabolic reaction does not occur, it is considered that other metabolic reactions are less likely to occur. Of course, it cannot be said that another metabolic reaction does not occur, but even in a case where another metabolic reaction occurs, it is considered that the influence degree of the metabolic reaction on the toxicity evaluation result is low. Therefore, by stopping the reaction prediction processing as in the present example, unnecessary prediction processing can be omitted.

In addition, as an example, in the output processing, the processor further outputs the metabolic process information indicating the metabolic reaction process. By outputting the metabolic process information, the user can easily grasp the evaluation result.

In an example, the in vitro toxicity evaluation test is an evaluation test for mutagenicity, sensitization, or irritation. The evaluation test of mutagenicity, sensitization, or irritation is an in vitro toxicity evaluation test that is generally used for toxicity evaluation of a chemical substance, and the toxicity evaluation device according to the technique of the present disclosure can be suitably applied.

Hereinafter, the rule data 21 including the compatibility rule will be described in more detail, and the terms used in the rule data 21 will be described in more detail.

First, as described above, the "compatibility rule suitable for an in vitro toxicity evaluation test" is a rule for allowing progression of metabolic reaction prediction to be compatible with an in vitro toxicity evaluation test based on the evaluation result obtained in an actual in vitro toxicity evaluation test. The compatibility rule includes, for example, a rule obtained from a relationship between a structure of a compound that has been subjected to toxicity evaluation in a past in vitro toxicity evaluation test and a metabolic mode estimated from a toxicity determination result.

The compatibility rule will be described with reference to a case where the in vitro toxicity test as a target is the Ames test. The Ames test is a test for evaluating the mutagenicity of a test substance and a metabolically activated product thereof, using five strains (TA100, TA1535, WP2uvrA, TA1537, and TA98) having different phenotypes, amino acid requirements, membrane permeabilities, drug resistances, and bacterial species (Salmonella or Escherichia coli). The mutagenicity evaluation of the test substance is performed by adding a test solution containing the test substance to a strain, culturing the strain, and then observing the resulting mutant colonies. The mutagenicity evaluation of the metabolically activated product is performed by carrying out the similar test using a strain to which a liver extract of a rat or a hamster and a coenzyme are added.

In the Ames test system, the progression of the metabolic reaction of the compound is defined by test-specific conditions such as a metabolic enzyme of a strain used for evaluation, a metabolic enzyme contained in a liver extract of a rat or a hamster, culture conditions, salts brought in from the compound, and a pH of the test system. On the other hand, among all chemical reactions estimated from the chemical structure of the test substance, there are also reactions that are unlikely to occur or do not occur in the Ames test, such as an enzyme reaction that is not present in the above-described test system. For example, there are many types of cytochrome P450, which is a metabolic enzyme, but also includes enzymes that do not function in the Ames test.

Therefore, in the prediction processing by the toxicity evaluation device of the present disclosure, a compatibility rule is set, for example, such that a reaction that is likely to occur in the Ames test is preferentially proceeded, and the priority of a reaction that is unlikely to occur or do not occur is lowered or the metabolic prediction is not proceeded. In a case where the metabolic reaction prediction is executed according to the compatibility rule set in this way, it is possible to predict the toxicity in the Ames test with high accuracy.

In addition, the reaction precondition, the reaction stop condition, the priority, and the like, which are included in the rule data 21, are as follows in more detail.

The reaction precondition is a precondition for a specific reaction to occur in the in vitro toxicity evaluation test. Examples of the reaction precondition include that the compound has a chemical structure necessary for a specific reaction to occur, that the compound has a value of a physical and chemical parameter that can cause a specific reaction to occur, and the like. In a case where the compound satisfies any of the reaction preconditions, the metabolic reaction prediction can be proceeded, and in a case where the compound does not satisfy any of the reaction preconditions, the metabolic reaction prediction can be terminated. The reaction precondition may include a condition related to a known chemical reaction, or may include a compatibility rule obtained from the in vitro toxicity evaluation test result.

Examples of the compatibility rule which is a reaction precondition include the following rules. In a case where the compound A has the partial structure a1 that is susceptible to the metabolic reaction X but has the other partial structure a2 and does not undergo the metabolic reaction X in the in vitro toxicity evaluation test, it is possible to set, as a reaction precondition for metabolic reaction X, that the compound has the partial structure a1 and does not have the partial structure a2.

The reaction stop condition is a condition under which the metabolic reaction is stopped in the in vitro toxicity evaluation test. That is, the reaction stop condition is a condition for predicting that a subsequent reaction does not occur with respect to a compound that is subject to a reaction prediction. Examples of the reaction stop condition include that the compound has a chemical structure necessary for stopping a certain reaction. In a case where the compound satisfies any of the reaction stop conditions, the metabolic reaction prediction can be terminated, and in a case where the compound does not satisfy any of the reaction stop conditions, the metabolic reaction prediction can proceed.

Examples of the compatibility rule which is a reaction stop condition include the following rules. In a case where, since the compound A has the chemical structure a3, subsequent reactions do not occur in the in vitro toxicity evaluation test, it is possible to set that the compound has the chemical structure a3 as a reaction stop condition. Examples of the cause of the lack of subsequent reaction in the in vitro toxicity evaluation test include a case where the compound reacts with DNA or protein due to having a specific chemical structure, thereby preventing further reactions from occurring.

The priority is a determination of an order for preferentially handling a metabolic reaction that is likely to occur in the in vitro toxicity evaluation test. For example, the priority is determined in ascending order by an integer (1, 2, 3, ... , and the like) with 1 as the highest order in order of priority from the most prioritized condition in the rule data. Of course, the numerical value of the priority may be determined in descending order such that the numerical value is a large number in a case where the priority is high.

In addition, in the rule data 21, the priority is given for each type of metabolic reaction. The "type of metabolic reaction" in this case refers to a type of chemical reaction that occurs with respect to a partial structure of a certain compound. That is, one type of chemical reaction occurring with respect to one type of partial structure is referred to as "one type of metabolic reaction". Even in a case where the partial structure of the compound and the chemical reaction are the same, there may be a difference in the mode of progression of the reaction depending on other partial structures of the compound. In such a case, each reaction is regarded as a different type of metabolic reaction.

Examples of the reaction by the metabolic enzyme include a reaction by a metabolic enzyme that is intrinsic to cells (for example, mammalian cells such as human cells, or bacterial cells) used in the in vitro toxicity test, or a reaction by other metabolic enzymes used in the in vitro toxicity test (for example, metabolic enzymes contained in a liver extract of a rat or hamster in the Ames test).

Examples of the reaction by a non-enzymatic reaction include an oxidation reaction, a hydrolysis reaction, a hydrogen shift reaction, and a decarboxylation reaction, which are not by an enzymatic reaction.

The compatibility rule that is the priority can be set as follows, for example. The reaction pathway and the reaction rate are estimated from the information obtained from the in vitro toxicity evaluation test, the simulation result of the metabolic reaction performed by mixing the metabolic activating enzyme in the in vitro toxicity evaluation test, and the like. Examples of the information obtained from the in vitro toxicity evaluation test include the presence or absence of metabolic enzymes in cells used in the test; a relationship between the concentration of a compound in cells used in the test and the toxicity strength (for example, the number of mutant colonies); a change in toxicity depending on the presence or absence of metabolic enzymes; and a mode of expression of toxicity of a known compound (for example, expression of toxicity due to covalent bonding between a compound and DNA or a protein, expression of toxicity due to intercalation of a compound between DNA base pairs, and the like). The simulation result of the metabolic reaction is, for example, a result of the metabolic reaction based on a test performed in a test tube. For example, the metabolic activating enzyme is mixed with a compound, and the compound is sampled over time to analyze a decrease in the amount of the compound, an increase in the amount of the degradation product, and the like. The reaction rate can be estimated by measuring the reduction rate (for example, half-life) of the compound. In addition, the reaction pathway can be estimated by performing structural analysis of the degradation product.

The metabolic activating enzyme in the in vitro toxicity evaluation test is, for example, a metabolic enzyme contained in a liver extract of a rat or a hamster, or the like. The priority is set based on the estimated reaction pathway and reaction rate. A rule is set for allowing progression of metabolic reaction prediction in descending order of priority.

In addition, the compatibility rule includes a rule obtained from a relationship between a structure of a compound that has been subjected to toxicity evaluation in a past in vitro toxicity evaluation test and a metabolic mode estimated from a toxicity determination result. The "metabolic mode" of a compound refers to a mode of a metabolic reaction including which metabolic reaction the compound undergoes and whether or not the compound undergoes a specific metabolic reaction.

For example, in a case where it is known that the partial structure a1 undergoes the metabolic reaction X, and the compound A having the partial structure a1 and the partial structure a2 does not undergo the metabolic reaction X in the in vitro toxicity evaluation test, it is presumed that the metabolic reaction X of the partial structure a1 is hindered by the presence of the partial structure a2. Therefore, it is presumed that the fact that the partial structure a1 is present and the partial structure a2 is not present is a precondition for the metabolic reaction X.

Whether or not the compound has undergone a specific metabolic reaction in the in vitro toxicity evaluation test may be estimated, for example, by the result of the toxicity determination. For example, in a case where it is known that a specific compound or partial structure exhibits toxicity in an in vitro toxicity evaluation test, it can be presumed that a compound which does not exhibit toxicity in the in vitro toxicity evaluation test has not been transformed into the compound or partial structure exhibiting toxicity.

By creating the compatibility rule based on the above-described information, it is possible to create the rule data 21 that is more suitable for the in vitro toxicity evaluation test. The compatibility rule created based on such information may be a reaction precondition, a reaction stop condition, or a priority.

Tables 1 and 2 below show more detailed examples of the rule data 21 shown in FIG. 4.

**[Table 1]**

| Item no. | Reaction information | | Reaction stop condition | Predicted reaction | Accumulation of reactant | Priority |
|---|---|---|---|---|---|---|
| | Reaction name | Reaction precondition | | | | |
| 1 | Reduction of nitro group | Ntrobenzene methyl substitution (excluding monomethyl substitution) | None | Nitro group → nitroso group | N | 2 |
| 2 | Reduction of nitroso group | Having nitroso group | None | Nitroso group → hydroxyamino group | N | 2 |
| 3 | Metabolic activation of hydroxyamino group | Having hydroxyamino group | None | Hydroxyamino group → nitrenium ion | N | 2 |
| 4 | | Presence of nitrenium ion | Nitrenium | Stop reaction | Y | 1 |
| 5 | Oxidation of alcohol | Having primary hydroxy group O or N is not substituted at C adjacent to C substituted with hydroxy group | None | Hydroxyl group → formyl group | Y | 4 |
| 6 | O-dealkylation | Having methoxy group-substituted or ethoxy group substituted benzene ring | None | Methoxy group or ethoxy group → hydroxyl group | N | 5 |
| 7 | Hydrogenation at allylic position | Having allyl group not adjacent to N, O, or S | None | Allyl group → substitution of hydroxy group at allyl position | N | 6 |
| 8 | Hydrogenation at α-position of aromatic ring | CH₂ adjacent to benzene ring, which is not adjacent to O or C, and whose adjacent atom is not bonded to C substituted with O or carbonyl | None | Benzyl group → substitution of hydroxy group at benzyl position | N | 3 |
| 9 | N-oxidation | Methoxyaniline, ethoxyaniline | None | Amino group → hydroxyamino group | N | 2 |
| 10 | N-oxidation | Aromatic acetamide (hydroxyl group is not present at o-position of amide group) | None | Acetylamino group → substitution of hydroxy group at N of acetylamino group | N | 3 |
| 11 | N-oxidation | o-halogenoaniline (two halogen substitutions or one substitute is alkyl substitution) | None | Amino group → hydroxyamino group | N | 2 |

**[Table 2]**

| Item no. | Reaction information | | Reaction stop condition | Predicted reaction | Accumulation of reactant | Priority |
|---|---|---|---|---|---|---|
| | Reaction name | Reaction precondition | | | | |
| 12 | N-dealkylation | Nitrosamine | None | Nitrosamine → substitution of hydroxyl group at carbon atom adjacent to proximal N of nitrosamine | N | 5 |
| 13 | N-dealkylation | Structure in which carbon atom adjacent to proximal N of nitrosamine is substituted with hydroxyl group | None | Substitution of hydroxyl group at carbon atom adjacent to proximal N of nitrosamine → structure in which electron transfer has occurred and the position of a double bond has changed | N | 3 |
| 14 | N-dealkylation | Structure in which hydroxyl group is substituted at carbon atom adjacent to proximal N of nitrosamine, and in which electron transfer has occurred to give N=N-[O-] | None | Structure in which electron transfer has occurred and the position of a double bond has changed → being decomposed to generate alkyl diazonium compound | N | 3 |
| 15 | N-dealkylation | Alkyl diazonium | None | Alkyl diazonium → alkyl-substituted carbocation formed by nitrogen elimination | N | 2 |
| 16 | N-dealkylation | Alkyl-substituted carbocation | Alkyl-substituted carbocation | Stop reaction | Y | 1 |
| 17 | Oxidative desulfurization | Having P=S | None | P=S → P=O | N | 5 |
| 18 | Hydrogenation of aromatic ring | Monosubstituted benzene | None | Hydroxylation at 4-position of monosubstituted benzene | Y | 7 |
| 19 | Epoxidation of aromatic ring | Phenanthrene | None | Phenanthrene → epoxidation at 1,2-position of phenanthrene | N | 3 |
| 20 | Ring opening of epoxide | Phenanthrene in which 1 and 2 positions are epoxidized | None | Phenanthrene in which 1 and 2 positions are epoxidized → phenanthrene having hydroxyl groups at positions 1 and 2 | N | 3 |
| 21 | Epoxidation of aromatic ring | Phenanthrene having hydroxyl groups at positions 1 and 2 | None | Phenanthrene having hydroxyl groups at positions 1 and 2 → epoxidation at 3,4-position of phenanthrene | N | 3 |
| 22 | Hydrolysis of amide | Formamide substituted with benzene ring | None | Formamide → formation of formaldehyde and amino group | N | 4 |

The "reaction name" in the above table is a reaction name used for convenience to indicate the type of the reaction, and is displayed on the user interface, for example. In the rule data in the above table, "reaction information" including "reaction name" and "reaction precondition" is set.

In the item no. 1 of the rule data in the above table, it is defined that the compound has a nitrobenzene methyl-substituted structure (excluding the monomethyl substitution) in the molecule as the reaction precondition. Assuming that the nitro group is reduced in monomethylnitrobenzene, nitrenium ions are generated, and a positive determination is made in the in vitro toxicity evaluation test, but in reality, a negative determination is obtained. Therefore, it can be presumed that the nitro group is not reduced in monomethylnitrobenzene. The present reaction precondition is a compatibility rule.

In item no. 10 of the rule data in the above table, as a reaction precondition, it is specified that the compound is aromatic acetamide and there is no hydroxyl group at the ortho position of the amide group. In general, it has been reported in a paper that oxidation occurs on a nitrogen atom in aromatic acetamide. On the other hand, in a case of assuming that an oxidation reaction occurs on a nitrogen atom in aromatic acetamide having a hydroxyl group at the ortho position of an amide group, a positive determination should be made in the in vitro toxicity evaluation test, but a negative determination is actually obtained. Therefore, it can be presumed that an oxidation reaction does not occur on the nitrogen atom in the aromatic acetamide having a hydroxyl group at the ortho position of the amide group. The present reaction precondition is a compatibility rule.

In item no. 11 of the rule data in the table above, as the reaction precondition, it is specified that the compound is o-halogenoaniline and has two halogen substitutions or has one halogen substitution and one alkyl substitution. In general, it is known that aniline generates a nitrenium ion that exhibits toxicity in a case where an amino group is oxidized. On the other hand, in a case of assuming that the amino group is oxidized in the o-halogenoaniline, a positive determination should be made in the in vitro toxicity evaluation test, but a negative determination is actually obtained except for a case where the o-halogenoaniline has two halogen substitutions or one halogen substitution and one alkyl substitution. Therefore, it can be presumed that the oxidation of the amino group occurs only in a case where the o-halogenoaniline has two halogen substitutions or one halogen substitution and one alkyl substitution. The present reaction precondition is a compatibility rule.

Item nos. 12 to 16 of the rules data in the above table are reactions that occur sequentially as a metabolic reaction of nitrosamine, and N-dealkylation occurs in the entire item nos. 12 to 16. Therefore, "N-dealkylation" is added as a "reaction name" for convenience.

Hereinafter, the toxicity evaluation device according to the present disclosure will be described with reference to specific examples. Provided that the embodiments of the present disclosure are not limited to the following specific examples.

A compatibility rule is created based on the Ames test results of the known compounds A to C. In the Ames test, TA100, TA1535, and WP2uvrA are strains capable of detecting a compound that generates a covalent bond with DNA and exhibits toxicity. In addition, TA98 and TA1537 are strains capable of detecting a compound exhibiting toxicity due to intercalation between base pairs formed of a double helix of DNA. The structures of the compounds A to C and the Ames test results obtained in the past are shown below. In the table, "without metabolic activation" is the test result obtained without adding the rat liver extract and the coenzyme, and "with metabolic activation" is the test result obtained by adding the rat liver extract and the coenzyme.

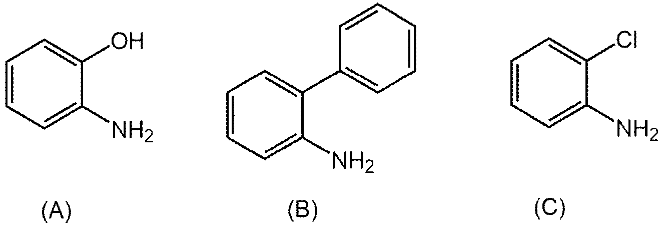

The Ames test results of the compound A are shown in the table below.

**[Table 3]**

| | Covalent bonding to DNA | | | Intercalation between base pairs | |
|---|---|---|---|---|---|
| | TA100 | TA1535 | WP2uvrA | TA98 | TA1537 |
| Without metabolic activation | No toxicity | No toxicity | No toxicity | No toxicity | No toxicity |
| With metabolic activation | Toxic | No toxicity | No toxicity | No toxicity | No toxicity |

The Ames test results of the compound B are shown in the table below.

**[Table 4]**

| | Covalent bonding to DNA | | | Intercalation between base pairs | |
|---|---|---|---|---|---|
| | TA100 | TA1535 | WP2uvrA | TA98 | TA1537 |
| Without metabolic activation | No toxicity | No toxicity | No toxicity | No toxicity | No toxicity |
| With metabolic activation | Toxic | No toxicity | No toxicity | No toxicity | No toxicity |

The Ames test results of the compound C are shown in the table below.

**[Table 5]**

| | Covalent bonding to DNA | | | Intercalation between base pairs | |
|---|---|---|---|---|---|
| | TA100 | TA1535 | WP2uvrA | TA98 | TA1537 |
| Without metabolic activation | No toxicity | No toxicity | No toxicity | No toxicity | No toxicity |
| With metabolic activation | No toxicity | No toxicity | No toxicity | No toxicity | No toxicity |

From the test results of the compounds A and B, it can be seen that both compounds A and B were changed to metabolites covalently bonded to DNA by the action of the metabolic enzyme in the Ames test. It is known that the aromatic amine is acted by a metabolic enzyme to form N-hydroxyarylamine, and then the N-hydroxyarylamine is acted by a metabolic enzyme to form a highly reactive nitrenium ion, which is covalently bonded to DNA. From the test results, it can be presumed that the compounds A and B generates nitrenium ions having a structure of covalently bonding to DNA by a metabolic reaction. On the other hand, since the compound C having the same partial structure does not generate a compound that forms a covalent bond with DNA even after the metabolic activation, it is presumed that the structural transformation to the nitrenium ion does not occur.

Based on the above information, a structural transformation reaction to nitrenium ion (hydroxylation of an amino group of an aromatic amine and metabolic activation of a hydroxylamino group) is set as "reaction information", and a condition that the aromatic amine is not substituted with chlorine at the ortho position of the amino group is set as "reaction precondition" as a condition for allowing such reaction to proceed.

In a case where a nitrenium ion is generated, the reaction rate with DNA is extremely high, whereby the reaction with DNA occurs preferentially over other metabolic reactions (for example, a reaction in which a hydroxyl group is introduced into a hydrogen-substituted portion of a benzene ring with the compound B). In addition, after the covalent bond with DNA, subsequent metabolic reactions do not occur. Therefore, the generation of nitrenium ions is set as the "reaction stop condition".

By collecting information in the above-described manner, rule data including "reaction precondition" and "reaction stop condition" including a compatibility rule suitable for an in vitro toxicity evaluation test is created. By creating rule data inductively from data obtained in an actual in vitro toxicity evaluation test, it is possible to perform toxicity evaluation suitable for an actual toxicity evaluation test.

For example, in a case where the condition that the aromatic amine is not substituted with chlorine at the ortho position of the amino group is not included in the "reaction precondition", the nitrenium ion is predicted as a metabolite of the compound C, and is erroneously determined to be positive in the toxicity evaluation, which may lower the specificity. In addition, in a case where the generation of nitrenium ions is not set as the "reaction stop condition", in the metabolic reaction prediction, for example, a reaction in which a hydroxyl group is introduced into a hydrogen-substituted portion of a benzene ring is predicted, and there is a possibility that the number of predicted metabolic products will increase.

In addition, in the present example, since the structure of the metabolic product is specified based on the information obtained from the in vitro toxicity evaluation test results, it is possible to suppress the prediction of a large number of metabolic products by predicting a metabolic reaction by an enzyme that does not actually work in the prediction processing.

In the present example, the rule data is applied to the structure input by the user, the structure is transformed through the metabolic reaction prediction, and the rule data is further applied to the generated metabolite to transform the structure, and this is repeated. In the in vivo reaction, it is considered that the environment in which the enzyme approaches is different between a case where the enzyme approaches the chemical substance to cause a first-stage structural transformation and a case of causing a second or higher stage structural transformation. Therefore, the range in which the structural transformation occurs is set to be different in the set priority order. Specifically, the structural transformation is caused to occur only for a reaction in which the difference in priority from the reaction having the highest priority is smaller than a value set in advance. As a result, in the second stage and subsequent stages of the structural transformation, the generation of a large number of structures of the metabolic product that may not actually occur is suppressed.

In the toxicity evaluation device in the present example, since a compatibility rule suitable for the in vitro toxicity evaluation test is defined, the prediction of the activity other than the toxicity detected in the in vitro toxicity evaluation test may be limited. However, the test substance that is negative based on the present method is predicted to be a chemical substance that does not express toxicity (for example, reactivity with DNA or protein) detected in the in vitro toxicity evaluation test. Therefore, it is considered that the specificity (that is, the proportion of negative test substances determined to be negative) for the toxicity evaluation of the in vitro toxicity evaluation test is maintained.

For example, in a case where the present toxicity evaluation device is used for screening for a useful compound having no toxicity, a low specificity may cause a large number of false positives, which may result in excessive exclusion of the useful compound from the candidates. Therefore, it is considered to be useful that the specificity is well maintained in the prediction of the toxicity evaluation.

In addition, in the above-described embodiment, the toxicity evaluation device 11 is shown in the form realized by a stand-alone personal computer, but may be realized in the form of a client-server type system. In this case, the toxicity evaluation device 11 is configured with a server computer that executes the prediction processing and the evaluation processing in response to the processing requests from the plurality of client terminals and delivers the final processing result 26 to each client terminal.

In addition, in the above-described embodiment, for example, as a hardware structure of the processor 60 that executes various types of processing such as the data acquisition processing, the prediction processing, and the evaluation processing, various processors shown below can be used. The various processors include, in addition to a CPU that is a general-purpose processor that executes software (program) to function as various processing units, a programmable logic device (PLD) of which a circuit configuration can be changed after manufacturing, such as a field-programmable gate array (FPGA), and a dedicated electric circuit that is a processor having a circuit configuration dedicatedly designed for executing specific processing, such as an application specific integrated circuit (ASIC).

Various types of processing described above may be executed by one of the various processors or may be executed by a combination of two or more processors (for example, a combination of a plurality of FPGAs or a CPU and an FPGA) of the same type or different types. A plurality of processing units may be configured by one processor. As an example in which the plurality of processing units are configured of one processor, there is a form in which a processor that realizes all functions of a system including the plurality of processing units by using one integrated circuit (IC) chip is used, such as a system on chip (SOC).

In this way, various processing units are configured by one or more of the above-described various processors as hardware structures.

Furthermore, the hardware structure of these various processors is, more specifically, an electric circuit (circuitry) in which circuit elements such as semiconductor elements are combined.

In addition, the technology of the present disclosure is applied to not only the operation program of the toxicity evaluation device 11 but also a non-transitory computer readable storage medium (a USB memory, a digital versatile disc (DVD)-read only memory (ROM), or the like) storing the operation program of the toxicity evaluation device 11.

The above-described contents and the above-shown contents are detailed descriptions for parts according to the technology of the present disclosure, and are merely examples of the technology of the present disclosure. For example, description related to the above configurations, functions, actions, and effects is description related to examples of configurations, functions, actions, and effects of the parts according to the disclosed technology. Thus, unnecessary parts may be removed, new elements may be added, or the parts may be replaced with each other in the content of description and the content of illustration shown above without departing from the gist of the disclosed technology. Particularly, description related to common technical knowledge or the like not required to be described for embodying the disclosed technology is omitted in the content of description and the content of illustration shown above in order to avoid complication and facilitate understanding of the parts according to the disclosed technology.

In the present specification, "A and/or B" is synonymous with "at least one of A or B" and "at least one selected from the group consisting of A and B". That is, "A and/or B" may mean only A, only B, or a combination of A and B. In the present specification, the same approach as "A and/or B" also applies to an expression of three or more matters connected with "and/or".

### (Appendices)

The following matters can also be understood by the above description of the present disclosure.
[1] A toxicity evaluation device comprising a processor,
   wherein the processor is configured to perform:
      data acquisition processing of acquiring test substance data specifying a test substance;
      prediction processing of predicting a metabolic reaction process of the test substance and a metabolite generated in the metabolic reaction process based on the test substance data;
      evaluation processing of acquiring an evaluation result by evaluating toxicity of at least one compound selected from the group consisting of the test substance and the metabolite; and
      output processing of outputting a processing result including the evaluation result,
   in the prediction processing, the processor is configured to predict the metabolic reaction process based on the test substance data and rule data specifying a rule in which a metabolic reaction proceeds, and
   the rule data includes a compatibility rule suitable for an in vitro toxicity evaluation test.
[2] The toxicity evaluation device according to [1],
   wherein the test substance data is selected from the group consisting of a structural formula of the test substance, a compositional formula of the test substance, and an amino acid sequence of the test substance.
[3] The toxicity evaluation device according to [1] or [2],
   wherein the rule data includes at least one condition selected from the group consisting of a reaction precondition that defines a premise under which a metabolic reaction occurs in the in vitro toxicity evaluation test, a reaction stop condition that defines a condition for stopping the metabolic reaction in the in vitro toxicity evaluation test, and a priority for preferentially handling a metabolic reaction that is likely to occur in the in vitro toxicity evaluation test, and the at least one condition selected from the group consisting of the reaction precondition, the reaction stop condition, and the priority includes the compatibility rule.
[4] The toxicity evaluation device according to [3],
   wherein the priority is a priority given for each type of the metabolic reaction, and the priority includes a priority determined based on at least one reaction rate selected from the group consisting of a reaction rate by a metabolic enzyme used in the in vitro toxicity evaluation test and a reaction rate by a non-enzymatic reaction.
[5] The toxicity evaluation device according to any one of [1] to [4],
   wherein the compatibility rule includes a rule obtained from a relationship between a structure of a compound that has been subjected to toxicity evaluation in a past in vitro toxicity evaluation test and a metabolic mode estimated from a toxicity determination result.
[6] The toxicity evaluation device according to any one of [1] to [5],
   wherein the prediction processing includes a reaction prediction processing of predicting a metabolic reaction that is repeatedly performed stepwise starting from the test substance and a metabolite generated by the metabolic reaction at each stage.
[7] The toxicity evaluation device according to [6],
   wherein the rule data is defined for each type of the metabolic reaction, and
   the processor is configured to execute, based on the rule data, the reaction prediction processing for each of the stages and each of the metabolic reactions.
[8] The toxicity evaluation device according to [6] or [7], in which, in the reaction prediction processing, a maximum value of the number of stages of a metabolic reaction that is repeatedly performed stepwise starting from the test substance or the metabolite is limited based on a structure of the test substance or the metabolite.
[9] The toxicity evaluation device according to any one of [6] to [8], in which, in the reaction prediction processing, in a case where a plurality of predictions of the metabolic reaction occurring in one of the stages starting from the test substance or the metabolite are possible, limitation on the number of predictions of the metabolic reaction in the stage is possible.
[10] The toxicity evaluation device according to [9],
   wherein the compatibility rule includes a priority for preferentially handling a metabolic reaction that is likely to occur in the in vitro toxicity evaluation test, and
   the limitation on the number of predictions is performed based on the priority.
[11] The toxicity evaluation device according to [10],
   wherein in a case where a stage of a metabolic reaction occurring in the test substance is defined as a first stage and a stage of a metabolic reaction occurring in the metabolite generated by the metabolic reaction in the first stage is defined as a second stage, the limitation on the number of predictions based on the priority is performed for the metabolic reactions in the second stage and subsequent stages.
[12] The toxicity evaluation device according to any one of [9] to [11],
   wherein the compatibility rule includes a reaction precondition that defines a premise under which a metabolic reaction occurs in the in vitro toxicity evaluation test, a reaction stop condition that defines a condition for stopping the metabolic reaction in the in vitro toxicity evaluation test, and a priority for preferentially handling a metabolic reaction that is likely to occur in the in vitro toxicity evaluation test, and
   the limitation on the number of predictions is performed by limiting a prediction of the metabolic reaction occurring in one of the stages to a metabolic reaction in which a difference in priority from a metabolic reaction having the highest priority among a plurality of metabolic reactions not satisfying the reaction stop condition and satisfying the reaction precondition is within a preset range.
[13] The toxicity evaluation device according to [12],
   wherein the preset range is less than 5.
[14] The toxicity evaluation device according to any one of [1] to [13],
   wherein, in the output processing, the processor is configured to further output metabolic process information specifying the metabolic reaction process.
[15] The toxicity evaluation device according to any one of [1] to [14],
   wherein the in vitro toxicity evaluation test is a test for evaluating mutagenicity, sensitization, or irritation.
[16] An operation method of a toxicity evaluation device including a processor,
   wherein the processor is configured to perform:
      data acquisition processing of acquiring test substance data specifying a test substance to be evaluated;
      prediction processing of predicting a metabolic reaction process of the test substance and a metabolite generated in the metabolic reaction process based on the test substance data;
      evaluation processing of acquiring an evaluation result by evaluating toxicity of at least one compound selected from the group consisting of the test substance and the metabolite; and
      output processing of outputting a processing result including the evaluation result,
   in the prediction processing, the processor is configured to predict the metabolic reaction process based on the test substance data and rule data specifying a rule in which a metabolic reaction proceeds, and
   the rule data includes a compatibility rule suitable for an in vitro toxicity evaluation test result.
[17] An operation program of a toxicity evaluation device for causing a computer to function as a toxicity evaluation device,
   wherein the operation program performs:
      data acquisition processing of acquiring test substance data specifying a test substance to be evaluated;
      prediction processing of predicting a metabolic reaction process of the test substance and a metabolite generated in the metabolic reaction process based on the test substance data;
      evaluation processing of acquiring an evaluation result by evaluating toxicity of at least one compound selected from the group consisting of the test substance and the metabolite; and
      output processing of outputting a processing result including the evaluation result,
   in the prediction processing, the metabolic reaction process is predicted based on the test substance data and rule data specifying a rule in which a metabolic reaction proceeds, and
   the rule data includes a compatibility rule suitable for an in vitro toxicity evaluation test result.

The disclosure of JP2023-059324 filed on March 31, 2023 is incorporated in the present specification by reference in its entirety. All documents, patent applications, and technical standards disclosed in the present specification are incorporated in the present specification by reference to the same extent as those in a case where each of the documents, patent applications, and technical standards are specifically and individually indicated to be incorporated by reference.

### Explanation of References

11: toxicity evaluation device
12: body part
13: display
14: input device
16: database (DB)
21: rule data for prediction processing
23: reference information for toxicity evaluation
25: test substance data
26: final processing result
50: storage
51: memory
52: CPU
53: communication unit
56: bus line
57: operation program
60: processor
66: table data

## Claims

1. A toxicity evaluation device comprising a processor,
wherein the processor is configured to perform:
data acquisition processing of acquiring test substance data specifying a test substance;
prediction processing of predicting a metabolic reaction process of the test substance and a metabolite generated in the metabolic reaction process based on the test substance data;
evaluation processing of acquiring an evaluation result by evaluating toxicity of at least one compound selected from the group consisting of the test substance and the metabolite; and
output processing of outputting a processing result including the evaluation result,
in the prediction processing, the processor is configured to predict the metabolic reaction process based on the test substance data and rule data specifying a rule in which a metabolic reaction proceeds, and
the rule data includes a compatibility rule suitable for an in vitro toxicity evaluation test.

2. The toxicity evaluation device according to claim 1,
wherein the test substance data is selected from the group consisting of a structural formula of the test substance, a compositional formula of the test substance, and an amino acid sequence of the test substance.

3. The toxicity evaluation device according to claim 1,
wherein the rule data includes at least one condition selected from the group consisting of a reaction precondition that defines a premise under which a metabolic reaction occurs in the in vitro toxicity evaluation test, a reaction stop condition that defines a condition for stopping the metabolic reaction in the in vitro toxicity evaluation test, and a priority for preferentially handling a metabolic reaction that is likely to occur in the in vitro toxicity evaluation test, and
the at least one condition selected from the group consisting of the reaction precondition, the reaction stop condition, and the priority includes the compatibility rule.

4. The toxicity evaluation device according to claim 3,
wherein the priority is a priority given for each type of the metabolic reaction, and
the priority includes a priority determined based on at least one reaction rate selected from the group consisting of a reaction rate by a metabolic enzyme used in the in vitro toxicity evaluation test and a reaction rate by a non-enzymatic reaction.

5. The toxicity evaluation device according to claim 1,
wherein the compatibility rule includes a rule obtained from a relationship between a structure of a compound that has been subjected to toxicity evaluation in a past in vitro toxicity evaluation test and a metabolic mode estimated from a toxicity determination result.

6. The toxicity evaluation device according to claim 1,
wherein the prediction processing includes a reaction prediction processing of predicting a metabolic reaction that is repeatedly performed stepwise starting from the test substance and a metabolite generated by the metabolic reaction at each stage.

7. The toxicity evaluation device according to claim 6,
wherein the rule data is defined for each type of the metabolic reaction, and
the processor is configured to execute, based on the rule data, the reaction prediction processing for each of the stages and each of the metabolic reactions.

8. The toxicity evaluation device according to claim 6,
wherein, in the reaction prediction processing, a maximum value of the number of stages of a metabolic reaction that is repeatedly performed stepwise starting from the test substance or the metabolite is limited based on a structure of the test substance or the metabolite.

9. The toxicity evaluation device according to claim 6,
wherein, in the reaction prediction processing, in a case where a plurality of predictions of the metabolic reaction occurring in one of the stages starting from the test substance or the metabolite are possible, limitation on the number of predictions of the metabolic reaction in the stage is possible.

10. The toxicity evaluation device according to claim 9,
wherein the compatibility rule includes a priority for preferentially handling a metabolic reaction that is likely to occur in the in vitro toxicity evaluation test, and
the limitation on the number of predictions is performed based on the priority.

11. The toxicity evaluation device according to claim 10,
wherein in a case where a stage of a metabolic reaction occurring in the test substance is defined as a first stage and a stage of a metabolic reaction occurring in the metabolite generated by the metabolic reaction in the first stage is defined as a second stage, the limitation on the number of predictions based on the priority is performed for the metabolic reactions in the second stage and subsequent stages.

12. The toxicity evaluation device according to claim 9,
wherein the compatibility rule includes a reaction precondition that defines a premise under which a metabolic reaction occurs in the in vitro toxicity evaluation test, a reaction stop condition that defines a condition for stopping the metabolic reaction in the in vitro toxicity evaluation test, and a priority for preferentially handling a metabolic reaction that is likely to occur in the in vitro toxicity evaluation test, and
the limitation on the number of predictions is performed by limiting a prediction of the metabolic reaction occurring in one of the stages to a metabolic reaction in which a difference in priority from a metabolic reaction having the highest priority among a plurality of metabolic reactions not satisfying the reaction stop condition and satisfying the reaction precondition is within a preset range.

13. The toxicity evaluation device according to claim 12,
wherein the preset range is less than 5.

14. The toxicity evaluation device according to claim 1,
wherein, in the output processing, the processor is configured to further output metabolic process information specifying the metabolic reaction process.

15. The toxicity evaluation device according to claim 1,
wherein the in vitro toxicity evaluation test is an evaluation test for mutagenicity, sensitization, or irritation.

16. An operation method of a toxicity evaluation device including a processor,
wherein the processor is configured to perform:
data acquisition processing of acquiring test substance data specifying a test substance to be evaluated;
prediction processing of predicting a metabolic reaction process of the test substance and a metabolite generated in the metabolic reaction process based on the test substance data;
evaluation processing of acquiring an evaluation result by evaluating toxicity of at least one compound selected from the group consisting of the test substance and the metabolite; and
output processing of outputting a processing result including the evaluation result,
in the prediction processing, the processor is configured to predict the metabolic reaction process based on the test substance data and rule data specifying a rule in which a metabolic reaction proceeds, and
the rule data includes a compatibility rule suitable for an in vitro toxicity evaluation test result.

17. An operation program of a toxicity evaluation device, for causing a computer to function as a toxicity evaluation device,
wherein the operation program performs:
data acquisition processing of acquiring test substance data specifying a test substance to be evaluated;
prediction processing of predicting a metabolic reaction process of the test substance and a metabolite generated in the metabolic reaction process based on the test substance data;
evaluation processing of acquiring an evaluation result by evaluating toxicity of at least one compound selected from the group consisting of the test substance and the metabolite; and
output processing of outputting a processing result including the evaluation result,
in the prediction processing, the metabolic reaction process is predicted based on the test substance data and rule data specifying a rule in which a metabolic reaction proceeds, and
the rule data includes a compatibility rule suitable for an in vitro toxicity evaluation test result.
